# EUROPEAN PATENT APPLICATION

(11) **EP 3 954 774 A1**
(43) Date of publication of application: **16.02.2022**
(21) Application number: 20786915.7
(22) Date of filing: 30.03.2020
(51) Int. Cl.: C12N 15/113, C12N 15/09, C12N 5/10, C12N 15/79, C12N 15/85, A61K 48/00

(54) **RNA SITE-DIRECTED EDITING USING ARTIFICIALLY CONSTRUCTED RNA EDITING ENZYMES AND RELATED USES**

(30) Priority: 08.04.2019 CN 201910277423
(71) Applicant: Shanghai Institute of Nutrition and Health, Chinese Academy of Sciences, Shanghai 200031 (CN)
(72) Inventor: WANG, Zefeng, Shanghai 200031 (CN); HAN, Wenjian, Shanghai 200031 (CN)
(74) Representative: Rondano, Davide
(86) International application number: PCT/CN2020/082183
(87) International publication number: WO 2020/207286

(57) **Abstract**

Disclosed are RNA site-directed editing using artificially constructed RNA editing enzymes and related uses. Provided is the fusion of an RNA recognition domain for binding RNA and a functional effector domain to form a new functional protein. The new functional protein specifically targets target RNA by means of the recognition domain and performs RNA editing using the effector domain.

SEQUENCE LISTING

<110>  SHANGHAI INSTITUTE OF NUTRITION AND HEALTH, CHINESE ACADEMY
        OF SCIENCES

<120>  RNA SITE-DIRECTED EDITING USING ARTIFICIALLY CONSTRUCTED RNA
       EDITING ENZYMES AND RELATED USES

<130>  P2021-2234

<150>  CN201910277423 .0
<151>  2019-04-08

<160>  14

<170>  PatentIn version 3.5

<210>  1
<211>  349
<212>  PRT
<213>  artificial sequence

<220>
<223> RNA recognition domain

<400>  1

Gly Arg Ser Arg Leu Leu Glu Asp Phe Arg Asn Asn Arg Tyr Pro Asn
1               5                   10                  15

Leu Gln Leu Arg Glu Ile Ala Gly His Ile Met Glu Phe Ser Gln Asp
            20                  25                  30

Gln His Gly Ser Arg Phe Ile Gln Leu Lys Leu Glu Arg Ala Thr Pro
        35                  40                  45

Ala Glu Arg Gln Leu Val Phe Asn Glu Ile Leu Gln Ala Ala Tyr Gln
    50                  55                  60

Leu Met Val Asp Val Phe Gly Asn Tyr Val Ile Gln Lys Phe Phe Glu
65                  70                  75                  80

Phe Gly Ser Leu Glu Gln Lys Leu Ala Leu Ala Glu Arg Ile Arg Gly
                85                  90                  95

His Val Leu Ser Leu Ala Leu Gln Met Tyr Gly Cys Arg Val Ile Gln
            100                 105                 110

Lys Ala Leu Glu Phe Ile Pro Ser Asp Gln Gln Asn Glu Met Val Arg
        115                 120                 125

Glu Leu Asp Gly His Val Leu Lys Cys Val Lys Asp Gln Asn Gly Asn
    130                 135                 140

His Val Val Gln Lys Cys Ile Glu Cys Val Gln Pro Gln Ser Leu Gln
145                 150                 155                 160

Phe Ile Ile Asp Ala Phe Lys Gly Gln Val Phe Ala Leu Ser Thr His
                165                 170                 175

Pro Tyr Gly Cys Arg Val Ile Gln Arg Ile Leu Glu His Cys Leu Pro
            180                 185                 190

Asp Gln Thr Leu Pro Ile Leu Glu Glu Leu His Gln His Thr Glu Gln
        195                 200                 205

Leu Val Gln Asp Gln Tyr Gly Asn Tyr Val Ile Gln His Val Leu Glu
    210                 215                 220

His Gly Arg Pro Glu Asp Lys Ser Lys Ile Val Ala Glu Ile Arg Gly
225                 230                 235                 240

Asn Val Leu Val Leu Ser Gln His Lys Phe Ala Ser Asn Val Val Glu
                245                 250                 255

Lys Cys Val Thr His Ala Ser Arg Thr Glu Arg Ala Val Leu Ile Asp
            260                 265                 270

Glu Val Cys Thr Met Asn Asp Gly Pro His Ser Ala Leu Tyr Thr Met
        275                 280                 285

Met Lys Asp Gln Tyr Ala Asn Tyr Val Val Gln Lys Met Ile Asp Val
    290                 295                 300

Ala Glu Pro Gly Gln Arg Lys Ile Val Met His Lys Ile Arg Pro His
305                 310                 315                 320

Ile Ala Thr Leu Arg Lys Tyr Thr Tyr Gly Lys His Ile Leu Ala Lys
                325                 330                 335

Leu Glu Lys Tyr Tyr Met Lys Asn Gly Val Asp Leu Gly
            340                 345

<210>  2
<211>  429
<212>  PRT
<213>  artificial sequence

<220>
<223> effector domain

<400>  2

Lys Ala Glu Arg Met Gly Phe Thr Glu Val Thr Pro Val Thr Gly Ala

1               5                   10                  15

Ser Leu Arg Arg Thr Met Leu Leu Leu Ser Arg Ser Pro Glu Ala Gln
            20                  25                  30

Pro Lys Thr Leu Pro Leu Thr Gly Ser Thr Phe His Asp Gln Ile Ala
        35                  40                  45

Met Leu Ser His Arg Cys Phe Asn Thr Leu Thr Asn Ser Phe Gln Pro
    50                  55                  60

Ser Leu Leu Gly Arg Lys Ile Leu Ala Ala Ile Ile Met Lys Lys Asp
65                  70                  75                  80

Ser Glu Asp Met Gly Val Val Val Ser Leu Gly Thr Gly Asn Arg Cys
                85                  90                  95

Val Lys Gly Asp Ser Leu Ser Leu Lys Gly Glu Thr Val Asn Asp Cys
            100                 105                 110

His Ala Glu Ile Ile Ser Arg Arg Gly Phe Ile Arg Phe Leu Tyr Ser
        115                 120                 125

Glu Leu Met Lys Tyr Asn Ser Gln Thr Ala Lys Asp Ser Ile Phe Glu
    130                 135                 140

Pro Ala Lys Gly Gly Glu Lys Leu Gln Ile Lys Lys Thr Val Ser Phe
145                 150                 155                 160

His Leu Tyr Ile Ser Thr Ala Pro Cys Gly Asp Gly Ala Leu Phe Asp
                165                 170                 175

Lys Ser Cys Ser Asp Arg Ala Met Glu Ser Thr Glu Ser Arg His Tyr
            180                 185                 190

Pro Val Phe Glu Asn Pro Lys Gln Gly Lys Leu Arg Thr Lys Val Glu
        195                 200                 205

Asn Gly Glu Gly Thr Ile Pro Val Glu Ser Ser Asp Ile Val Pro Thr
    210                 215                 220

Trp Asp Gly Ile Arg Leu Gly Glu Arg Leu Arg Thr Met Ser Cys Ser
225                 230                 235                 240

Asp Lys Ile Leu Arg Trp Asn Val Leu Gly Leu Gln Gly Ala Leu Leu
                245                 250                 255

Thr His Phe Leu Gln Pro Ile Tyr Leu Lys Ser Val Thr Leu Gly Tyr
            260                 265                 270

Leu Phe Ser Gln Gly His Leu Thr Arg Ala Ile Cys Cys Arg Val Thr
        275                 280                 285

Arg Asp Gly Ser Ala Phe Glu Asp Gly Leu Arg His Pro Phe Ile Val
    290                 295                 300

Asn His Pro Lys Val Gly Arg Val Ser Ile Tyr Asp Ser Lys Arg Gln
305                 310                 315                 320

Ser Gly Lys Thr Lys Glu Thr Ser Val Asn Trp Cys Leu Ala Asp Gly
                325                 330                 335

Tyr Asp Leu Glu Ile Leu Asp Gly Thr Arg Gly Thr Val Asp Gly Pro
            340                 345                 350

Arg Asn Glu Leu Ser Arg Val Ser Lys Lys Asn Ile Phe Leu Leu Phe
        355                 360                 365

Lys Lys Leu Cys Ser Phe Arg Tyr Arg Arg Asp Leu Leu Arg Leu Ser
    370                 375                 380

Tyr Gly Glu Ala Lys Lys Ala Ala Arg Asp Tyr Glu Thr Ala Lys Asn
385                 390                 395                 400

Tyr Phe Lys Lys Gly Leu Lys Asp Met Gly Tyr Gly Asn Trp Ile Ser
                405                 410                 415

Lys Pro Gln Glu Glu Lys Asn Phe Tyr Leu Cys Pro Val
            420                 425

<210>  3
<211>  440
<212>  PRT
<213>  artificial sequence

<220>
<223>  the effector domain of ADAR2-isoform1

<400>  3

Asp Gln Thr Pro Ser Arg Gln Pro Ile Pro Ser Glu Gly Leu Gln Leu
1               5                   10                  15

His Leu Pro Gln Val Leu Ala Asp Ala Val Ser Arg Leu Val Leu Gly
            20                  25                  30

Lys Phe Gly Asp Leu Thr Asp Asn Phe Ser Ser Pro His Ala Arg Arg

35                  40                  45

Lys Val Leu Ala Gly Val Val Met Thr Thr Gly Thr Asp Val Lys Asp
    50                  55                  60

Ala Lys Val Ile Ser Val Ser Thr Gly Thr Lys Cys Ile Asn Gly Glu
65                  70                  75                  80

Tyr Met Ser Asp Arg Gly Leu Ala Leu Asn Asp Cys His Ala Glu Ile
                85                  90                  95

Ile Ser Arg Arg Ser Leu Leu Arg Phe Leu Tyr Thr Gln Leu Glu Leu
            100                 105                 110

Tyr Leu Asn Asn Lys Asp Asp Gln Lys Arg Ser Ile Phe Gln Lys Ser
        115                 120                 125

Glu Arg Gly Gly Phe Arg Leu Lys Glu Asn Val Gln Phe His Leu Tyr
    130                 135                 140

Ile Ser Thr Ser Pro Cys Gly Asp Ala Arg Ile Phe Ser Pro His Glu
145                 150                 155                 160

Pro Ile Leu Glu Gly Ser Arg Ser Tyr Thr Gln Ala Gly Val Gln Trp
                165                 170                 175

Cys Asn His Gly Ser Leu Gln Pro Arg Pro Pro Gly Leu Leu Ser Asp
            180                 185                 190

Pro Ser Thr Ser Thr Phe Gln Gly Ala Gly Thr Thr Glu Pro Ala Asp
        195                 200                 205

Arg His Pro Asn Arg Lys Ala Arg Gly Gln Leu Arg Thr Lys Ile Glu
    210                 215                 220

Ser Gly Glu Gly Thr Ile Pro Val Arg Ser Asn Ala Ser Ile Gln Thr
225                 230                 235                 240

Trp Asp Gly Val Leu Gln Gly Glu Arg Leu Leu Thr Met Ser Cys Ser
                245                 250                 255

Asp Lys Ile Ala Arg Trp Asn Val Val Gly Ile Gln Gly Ser Leu Leu
            260                 265                 270

Ser Ile Phe Val Glu Pro Ile Tyr Phe Ser Ser Ile Ile Leu Gly Ser
        275                 280                 285

Leu Tyr His Gly Asp His Leu Ser Arg Ala Met Tyr Gln Arg Ile Ser
    290                 295                 300

Asn Ile Glu Asp Leu Pro Pro Leu Tyr Thr Leu Asn Lys Pro Leu Leu
305                 310                 315                 320

Ser Gly Ile Ser Asn Ala Glu Ala Arg Gln Pro Gly Lys Ala Pro Asn
                325                 330                 335

Phe Ser Val Asn Trp Thr Val Gly Asp Ser Ala Ile Glu Val Ile Asn
            340                 345                 350

Ala Thr Thr Gly Lys Asp Glu Leu Gly Arg Ala Ser Arg Leu Cys Lys
        355                 360                 365

His Ala Leu Tyr Cys Arg Trp Met Arg Val His Gly Lys Val Pro Ser
    370                 375                 380

His Leu Leu Arg Ser Lys Ile Thr Lys Pro Asn Val Tyr His Glu Ser
385                 390                 395                 400

Lys Leu Ala Ala Lys Glu Tyr Gln Ala Ala Lys Ala Arg Leu Phe Thr
                405                 410                 415

Ala Phe Ile Lys Ala Gly Leu Gly Ala Trp Val Glu Lys Pro Thr Glu
            420                 425                 430

Gln Asp Gln Phe Ser Leu Thr Pro
        435                 440

<210>  4
<211>  400
<212>  PRT
<213>  artificial sequence

<220>
<223>  the ADAR2-isoform2 effector domain

<400>  4

Asp Gln Thr Pro Ser Arg Gln Pro Ile Pro Ser Glu Gly Leu Gln Leu
1               5                   10                  15

His Leu Pro Gln Val Leu Ala Asp Ala Val Ser Arg Leu Val Leu Gly
            20                  25                  30

Lys Phe Gly Asp Leu Thr Asp Asn Phe Ser Ser Pro His Ala Arg Arg
        35                  40                  45

Lys Val Leu Ala Gly Val Val Met Thr Thr Gly Thr Asp Val Lys Asp

50                  55                  60

Ala Lys Val Ile Ser Val Ser Thr Gly Thr Lys Cys Ile Asn Gly Glu
65                  70                  75                  80

Tyr Met Ser Asp Arg Gly Leu Ala Leu Asn Asp Cys His Ala Glu Ile
                85                  90                  95

Ile Ser Arg Arg Ser Leu Leu Arg Phe Leu Tyr Thr Gln Leu Glu Leu
            100                 105                 110

Tyr Leu Asn Asn Lys Asp Asp Gln Lys Arg Ser Ile Phe Gln Lys Ser
        115                 120                 125

Glu Arg Gly Gly Phe Arg Leu Lys Glu Asn Val Gln Phe His Leu Tyr
    130                 135                 140

Ile Ser Thr Ser Pro Cys Gly Asp Ala Arg Ile Phe Ser Pro His Glu
145                 150                 155                 160

Pro Ile Leu Glu Glu Pro Ala Asp Arg His Pro Asn Arg Lys Ala Arg
                165                 170                 175

Gly Gln Leu Arg Thr Lys Ile Glu Ser Gly Glu Gly Thr Ile Pro Val
            180                 185                 190

Arg Ser Asn Ala Ser Ile Gln Thr Trp Asp Gly Val Leu Gln Gly Glu
        195                 200                 205

Arg Leu Leu Thr Met Ser Cys Ser Asp Lys Ile Ala Arg Trp Asn Val
    210                 215                 220

Val Gly Ile Gln Gly Ser Leu Leu Ser Ile Phe Val Glu Pro Ile Tyr
225                 230                 235                 240

Phe Ser Ser Ile Ile Leu Gly Ser Leu Tyr His Gly Asp His Leu Ser
                245                 250                 255

Arg Ala Met Tyr Gln Arg Ile Ser Asn Ile Glu Asp Leu Pro Pro Leu
            260                 265                 270

Tyr Thr Leu Asn Lys Pro Leu Leu Ser Gly Ile Ser Asn Ala Glu Ala
        275                 280                 285

Arg Gln Pro Gly Lys Ala Pro Asn Phe Ser Val Asn Trp Thr Val Gly
    290                 295                 300

Asp Ser Ala Ile Glu Val Ile Asn Ala Thr Thr Gly Lys Asp Glu Leu
305                 310                 315                 320

Gly Arg Ala Ser Arg Leu Cys Lys His Ala Leu Tyr Cys Arg Trp Met
                325                 330                 335

Arg Val His Gly Lys Val Pro Ser His Leu Leu Arg Ser Lys Ile Thr
            340                 345                 350

Lys Pro Asn Val Tyr His Glu Ser Lys Leu Ala Ala Lys Glu Tyr Gln
        355                 360                 365

Ala Ala Lys Ala Arg Leu Phe Thr Ala Phe Ile Lys Ala Gly Leu Gly
    370                 375                 380

Ala Trp Val Glu Lys Pro Thr Glu Gln Asp Gln Phe Ser Leu Thr Pro
385                 390                 395                 400

<210>  5
<211>  199
<212>  PRT
<213>  artificial sequence

<220>
<223> APOBEC3A

<400>  5

Met Glu Ala Ser Pro Ala Ser Gly Pro Arg His Leu Met Asp Pro His
1               5                   10                  15

Ile Phe Thr Ser Asn Phe Asn Asn Gly Ile Gly Arg His Lys Thr Tyr
            20                  25                  30

Leu Cys Tyr Glu Val Glu Arg Leu Asp Asn Gly Thr Ser Val Lys Met
        35                  40                  45

Asp Gln His Arg Gly Phe Leu His Asn Gln Ala Lys Asn Leu Leu Cys
    50                  55                  60

Gly Phe Tyr Gly Arg His Ala Glu Leu Arg Phe Leu Asp Leu Val Pro
65                  70                  75                  80

Ser Leu Gln Leu Asp Pro Ala Gln Ile Tyr Arg Val Thr Trp Phe Ile
                85                  90                  95

Ser Trp Ser Pro Cys Phe Ser Trp Gly Cys Ala Gly Glu Val Arg Ala
            100                 105                 110

Phe Leu Gln Glu Asn Thr His Val Arg Leu Arg Ile Phe Ala Ala Arg

115                 120                 125

Ile Tyr Asp Tyr Asp Pro Leu Tyr Lys Glu Ala Leu Gln Met Leu Arg
    130                 135                 140

Asp Ala Gly Ala Gln Val Ser Ile Met Thr Tyr Asp Glu Phe Lys His
145                 150                 155                 160

Cys Trp Asp Thr Phe Val Asp His Gln Gly Cys Pro Phe Gln Pro Trp
                165                 170                 175

Asp Gly Leu Asp Glu His Ser Gln Ala Leu Ser Gly Arg Leu Arg Ala
            180                 185                 190

Ile Leu Gln Asn Gln Gly Asn
        195

<210>  6
<211>  7
<212>  PRT
<213>  artificial sequence

<220>
<223> the linker peptide

<400>  6

Glu Phe Thr Gly Asn Gly Ser
1               5

<210>  7
<211>  16
<212>  PRT
<213>  artificial sequence

<220>
<223> the linker peptide

<400>  7

Ser Gly Ser Glu Thr Pro Gly Thr Ser Glu Ser Ala Thr Pro Glu Ser
1               5                   10                  15

<210>  8
<211>  20
<212>  PRT
<213>  artificial sequence

<220>
<223> the linker peptide

<400>  8

Asp Gln Thr Pro Ser Arg Gln Pro Ile Pro Ser Glu Gly Leu Gln Leu
1               5                   10                  15

His Leu Pro Gln
            20

<210>  9
<211>  41
<212>  PRT
<213>  artificial sequence

<220>
<223> the linker peptide

<400>  9

Lys Ala Glu Arg Met Gly Phe Thr Glu Val Thr Pro Val Thr Gly Ala
1               5                   10                  15

Ser Leu Arg Arg Thr Met Leu Leu Leu Ser Arg Ser Pro Glu Ala Gln
            20                  25                  30

Pro Lys Thr Leu Pro Leu Thr Gly Ser
        35                  40

<210>  10
<211>  1226
<212>  PRT
<213>  artificial sequence

<220>
<223> ADAR1

<400>  10

Met Asn Pro Arg Gln Gly Tyr Ser Leu Ser Gly Tyr Tyr Thr His Pro
1               5                   10                  15

Phe Gln Gly Tyr Glu His Arg Gln Leu Arg Tyr Gln Gln Pro Gly Pro
            20                  25                  30

Gly Ser Ser Pro Ser Ser Phe Leu Leu Lys Gln Ile Glu Phe Leu Lys
        35                  40                  45

Gly Gln Leu Pro Glu Ala Pro Val Ile Gly Lys Gln Thr Pro Ser Leu
    50                  55                  60

Pro Pro Ser Leu Pro Gly Leu Arg Pro Arg Phe Pro Val Leu Leu Ala
65                  70                  75                  80

Ser Ser Thr Arg Gly Arg Gln Val Asp Ile Arg Gly Val Pro Arg Gly
                85                  90                  95

Val His Leu Arg Ser Gln Gly Leu Gln Arg Gly Phe Gln His Pro Ser

            100                 105                 110

Pro Arg Gly Arg Ser Leu Pro Gln Arg Gly Val Asp Cys Leu Ser Ser
        115                 120                 125

His Phe Gln Glu Leu Ser Ile Tyr Gln Asp Gln Glu Gln Arg Ile Leu
    130                 135                 140

Lys Phe Leu Glu Glu Leu Gly Glu Gly Lys Ala Thr Thr Ala His Asp
145                 150                 155                 160

Leu Ser Gly Lys Leu Gly Thr Pro Lys Lys Glu Ile Asn Arg Val Leu
                165                 170                 175

Tyr Ser Leu Ala Lys Lys Gly Lys Leu Gln Lys Glu Ala Gly Thr Pro
            180                 185                 190

Pro Leu Trp Lys Ile Ala Val Ser Thr Gln Ala Trp Asn Gln His Ser
        195                 200                 205

Gly Val Val Arg Pro Asp Gly His Ser Gln Gly Ala Pro Asn Ser Asp
    210                 215                 220

Pro Ser Leu Glu Pro Glu Asp Arg Asn Ser Thr Ser Val Ser Glu Asp
225                 230                 235                 240

Leu Leu Glu Pro Phe Ile Ala Val Ser Ala Gln Ala Trp Asn Gln His
                245                 250                 255

Ser Gly Val Val Arg Pro Asp Ser His Ser Gln Gly Ser Pro Asn Ser
            260                 265                 270

Asp Pro Gly Leu Glu Pro Glu Asp Ser Asn Ser Thr Ser Ala Leu Glu
        275                 280                 285

Asp Pro Leu Glu Phe Leu Asp Met Ala Glu Ile Lys Glu Lys Ile Cys
    290                 295                 300

Asp Tyr Leu Phe Asn Val Ser Asp Ser Ser Ala Leu Asn Leu Ala Lys
305                 310                 315                 320

Asn Ile Gly Leu Thr Lys Ala Arg Asp Ile Asn Ala Val Leu Ile Asp
                325                 330                 335

Met Glu Arg Gln Gly Asp Val Tyr Arg Gln Gly Thr Thr Pro Pro Ile
            340                 345                 350

Trp His Leu Thr Asp Lys Lys Arg Glu Arg Met Gln Ile Lys Arg Asn
        355                 360                 365

Thr Asn Ser Val Pro Glu Thr Ala Pro Ala Ala Ile Pro Glu Thr Lys
    370                 375                 380

Arg Asn Ala Glu Phe Leu Thr Cys Asn Ile Pro Thr Ser Asn Ala Ser
385                 390                 395                 400

Asn Asn Met Val Thr Thr Glu Lys Val Glu Asn Gly Gln Glu Pro Val
                405                 410                 415

Ile Lys Leu Glu Asn Arg Gln Glu Ala Arg Pro Glu Pro Ala Arg Leu
            420                 425                 430

Lys Pro Pro Val His Tyr Asn Gly Pro Ser Lys Ala Gly Tyr Val Asp
        435                 440                 445

Phe Glu Asn Gly Gln Trp Ala Thr Asp Asp Ile Pro Asp Asp Leu Asn
    450                 455                 460

Ser Ile Arg Ala Ala Pro Gly Glu Phe Arg Ala Ile Met Glu Met Pro
465                 470                 475                 480

Ser Phe Tyr Ser His Gly Leu Pro Arg Cys Ser Pro Tyr Lys Lys Leu
                485                 490                 495

Thr Glu Cys Gln Leu Lys Asn Pro Ile Ser Gly Leu Leu Glu Tyr Ala
            500                 505                 510

Gln Phe Ala Ser Gln Thr Cys Glu Phe Asn Met Ile Glu Gln Ser Gly
        515                 520                 525

Pro Pro His Glu Pro Arg Phe Lys Phe Gln Val Val Ile Asn Gly Arg
    530                 535                 540

Glu Phe Pro Pro Ala Glu Ala Gly Ser Lys Lys Val Ala Lys Gln Asp
545                 550                 555                 560

Ala Ala Met Lys Ala Met Thr Ile Leu Leu Glu Glu Ala Lys Ala Lys
                565                 570                 575

Asp Ser Gly Lys Ser Glu Glu Ser Ser His Tyr Ser Thr Glu Lys Glu
            580                 585                 590

Ser Glu Lys Thr Ala Glu Ser Gln Thr Pro Thr Pro Ser Ala Thr Ser
        595                 600                 605

Phe Phe Ser Gly Lys Ser Pro Val Thr Thr Leu Leu Glu Cys Met His
    610                 615                 620

Lys Leu Gly Asn Ser Cys Glu Phe Arg Leu Leu Ser Lys Glu Gly Pro
625                 630                 635                 640

Ala His Glu Pro Lys Phe Gln Tyr Cys Val Ala Val Gly Ala Gln Thr
                645                 650                 655

Phe Pro Ser Val Ser Ala Pro Ser Lys Lys Val Ala Lys Gln Met Ala
            660                 665                 670

Ala Glu Glu Ala Met Lys Ala Leu His Gly Glu Ala Thr Asn Ser Met
        675                 680                 685

Ala Ser Asp Asn Gln Pro Glu Gly Met Ile Ser Glu Ser Leu Asp Asn
    690                 695                 700

Leu Glu Ser Met Met Pro Asn Lys Val Arg Lys Ile Gly Glu Leu Val
705                 710                 715                 720

Arg Tyr Leu Asn Thr Asn Pro Val Gly Gly Leu Leu Glu Tyr Ala Arg
                725                 730                 735

Ser His Gly Phe Ala Ala Glu Phe Lys Leu Val Asp Gln Ser Gly Pro
            740                 745                 750

Pro His Glu Pro Lys Phe Val Tyr Gln Ala Lys Val Gly Gly Arg Trp
        755                 760                 765

Phe Pro Ala Val Cys Ala His Ser Lys Lys Gln Gly Lys Gln Glu Ala
    770                 775                 780

Ala Asp Ala Ala Leu Arg Val Leu Ile Gly Glu Asn Glu Lys Ala Glu
785                 790                 795                 800

Arg Met Gly Phe Thr Glu Val Thr Pro Val Thr Gly Ala Ser Leu Arg
                805                 810                 815

Arg Thr Met Leu Leu Leu Ser Arg Ser Pro Glu Ala Gln Pro Lys Thr
            820                 825                 830

Leu Pro Leu Thr Gly Ser Thr Phe His Asp Gln Ile Ala Met Leu Ser
        835                 840                 845

His Arg Cys Phe Asn Thr Leu Thr Asn Ser Phe Gln Pro Ser Leu Leu
    850                 855                 860

Gly Arg Lys Ile Leu Ala Ala Ile Ile Met Lys Lys Asp Ser Glu Asp
865                 870                 875                 880

Met Gly Val Val Val Ser Leu Gly Thr Gly Asn Arg Cys Val Lys Gly
                885                 890                 895

Asp Ser Leu Ser Leu Lys Gly Glu Thr Val Asn Asp Cys His Ala Glu
            900                 905                 910

Ile Ile Ser Arg Arg Gly Phe Ile Arg Phe Leu Tyr Ser Glu Leu Met
        915                 920                 925

Lys Tyr Asn Ser Gln Thr Ala Lys Asp Ser Ile Phe Glu Pro Ala Lys
    930                 935                 940

Gly Gly Glu Lys Leu Gln Ile Lys Lys Thr Val Ser Phe His Leu Tyr
945                 950                 955                 960

Ile Ser Thr Ala Pro Cys Gly Asp Gly Ala Leu Phe Asp Lys Ser Cys
                965                 970                 975

Ser Asp Arg Ala Met Glu Ser Thr Glu Ser Arg His Tyr Pro Val Phe
            980                 985                 990

Glu Asn Pro Lys Gln Gly Lys Leu Arg Thr Lys Val Glu Asn Gly Glu
        995                 1000                1005

Gly Thr Ile Pro Val Glu Ser Ser Asp Ile Val Pro Thr Trp Asp
    1010                1015                1020

Gly Ile Arg Leu Gly Glu Arg Leu Arg Thr Met Ser Cys Ser Asp
    1025                1030                1035

Lys Ile Leu Arg Trp Asn Val Leu Gly Leu Gln Gly Ala Leu Leu
    1040                1045                1050

Thr His Phe Leu Gln Pro Ile Tyr Leu Lys Ser Val Thr Leu Gly
    1055                1060                1065

Tyr Leu Phe Ser Gln Gly His Leu Thr Arg Ala Ile Cys Cys Arg
    1070                1075                1080

Val Thr Arg Asp Gly Ser Ala Phe Glu Asp Gly Leu Arg His Pro
    1085                1090                1095

Phe Ile Val Asn His Pro Lys Val Gly Arg Val Ser Ile Tyr Asp

1100                    1105                    1110

Ser Lys  Arg Gln Ser Gly Lys  Thr Lys Glu Thr Ser  Val Asn Trp
    1115                    1120                    1125

Cys Leu  Ala Asp Gly Tyr Asp  Leu Glu Ile Leu Asp  Gly Thr Arg
    1130                    1135                    1140

Gly Thr  Val Asp Gly Pro Arg  Asn Glu Leu Ser Arg  Val Ser Lys
    1145                    1150                    1155

Lys Asn  Ile Phe Leu Leu Phe  Lys Lys Leu Cys Ser  Phe Arg Tyr
    1160                    1165                    1170

Arg Arg  Asp Leu Leu Arg Leu  Ser Tyr Gly Glu Ala  Lys Lys Ala
    1175                    1180                    1185

Ala Arg  Asp Tyr Glu Thr Ala  Lys Asn Tyr Phe Lys  Lys Gly Leu
    1190                    1195                    1200

Lys Asp  Met Gly Tyr Gly Asn  Trp Ile Ser Lys Pro  Gln Glu Glu
    1205                    1210                    1215

Lys Asn  Phe Tyr Leu Cys Pro  Val
    1220                    1225

<210>  11
<211>  741
<212>  PRT
<213>  artificial sequence

<220>
<223>  ADAR2-isoform1

<400>  11

Met Asp Ile Glu Asp Glu Glu Asn Met Ser Ser Ser Ser Thr Asp Val
1               5                   10                  15

Lys Glu Asn Arg Asn Leu Asp Asn Val Ser Pro Lys Asp Gly Ser Thr
            20                  25                  30

Pro Gly Pro Gly Glu Gly Ser Gln Leu Ser Asn Gly Gly Gly Gly Gly
        35                  40                  45

Pro Gly Arg Lys Arg Pro Leu Glu Glu Gly Ser Asn Gly His Ser Lys
    50                  55                  60

Tyr Arg Leu Lys Lys Arg Arg Lys Thr Pro Gly Pro Val Leu Pro Lys
65                  70                  75                  80

Asn Ala Leu Met Gln Leu Asn Glu Ile Lys Pro Gly Leu Gln Tyr Thr
                85                  90                  95

Leu Leu Ser Gln Thr Gly Pro Val His Ala Pro Leu Phe Val Met Ser
            100                 105                 110

Val Glu Val Asn Gly Gln Val Phe Glu Gly Ser Gly Pro Thr Lys Lys
        115                 120                 125

Lys Ala Lys Leu His Ala Ala Glu Lys Ala Leu Arg Ser Phe Val Gln
    130                 135                 140

Phe Pro Asn Ala Ser Glu Ala His Leu Ala Met Gly Arg Thr Leu Ser
145                 150                 155                 160

Val Asn Thr Asp Phe Thr Ser Asp Gln Ala Asp Phe Pro Asp Thr Leu
                165                 170                 175

Phe Asn Gly Phe Glu Thr Pro Asp Lys Ala Glu Pro Pro Phe Tyr Val
            180                 185                 190

Gly Ser Asn Gly Asp Asp Ser Phe Ser Ser Ser Gly Asp Leu Ser Leu
        195                 200                 205

Ser Ala Ser Pro Val Pro Ala Ser Leu Ala Gln Pro Pro Leu Pro Val
    210                 215                 220

Leu Pro Pro Phe Pro Pro Pro Ser Gly Lys Asn Pro Val Met Ile Leu
225                 230                 235                 240

Asn Glu Leu Arg Pro Gly Leu Lys Tyr Asp Phe Leu Ser Glu Ser Gly
                245                 250                 255

Glu Ser His Ala Lys Ser Phe Val Met Ser Val Val Val Asp Gly Gln
            260                 265                 270

Phe Phe Glu Gly Ser Gly Arg Asn Lys Lys Leu Ala Lys Ala Arg Ala
        275                 280                 285

Ala Gln Ser Ala Leu Ala Ala Ile Phe Asn Leu His Leu Asp Gln Thr
    290                 295                 300

Pro Ser Arg Gln Pro Ile Pro Ser Glu Gly Leu Gln Leu His Leu Pro
305                 310                 315                 320

Gln Val Leu Ala Asp Ala Val Ser Arg Leu Val Leu Gly Lys Phe Gly

325                 330                 335

Asp Leu Thr Asp Asn Phe Ser Ser Pro His Ala Arg Arg Lys Val Leu
            340                 345                 350

Ala Gly Val Val Met Thr Thr Gly Thr Asp Val Lys Asp Ala Lys Val
        355                 360                 365

Ile Ser Val Ser Thr Gly Thr Lys Cys Ile Asn Gly Glu Tyr Met Ser
    370                 375                 380

Asp Arg Gly Leu Ala Leu Asn Asp Cys His Ala Glu Ile Ile Ser Arg
385                 390                 395                 400

Arg Ser Leu Leu Arg Phe Leu Tyr Thr Gln Leu Glu Leu Tyr Leu Asn
                405                 410                 415

Asn Lys Asp Asp Gln Lys Arg Ser Ile Phe Gln Lys Ser Glu Arg Gly
            420                 425                 430

Gly Phe Arg Leu Lys Glu Asn Val Gln Phe His Leu Tyr Ile Ser Thr
        435                 440                 445

Ser Pro Cys Gly Asp Ala Arg Ile Phe Ser Pro His Glu Pro Ile Leu
    450                 455                 460

Glu Gly Ser Arg Ser Tyr Thr Gln Ala Gly Val Gln Trp Cys Asn His
465                 470                 475                 480

Gly Ser Leu Gln Pro Arg Pro Pro Gly Leu Leu Ser Asp Pro Ser Thr
                485                 490                 495

Ser Thr Phe Gln Gly Ala Gly Thr Thr Glu Pro Ala Asp Arg His Pro
            500                 505                 510

Asn Arg Lys Ala Arg Gly Gln Leu Arg Thr Lys Ile Glu Ser Gly Glu
        515                 520                 525

Gly Thr Ile Pro Val Arg Ser Asn Ala Ser Ile Gln Thr Trp Asp Gly
    530                 535                 540

Val Leu Gln Gly Glu Arg Leu Leu Thr Met Ser Cys Ser Asp Lys Ile
545                 550                 555                 560

Ala Arg Trp Asn Val Val Gly Ile Gln Gly Ser Leu Leu Ser Ile Phe
                565                 570                 575

Val Glu Pro Ile Tyr Phe Ser Ser Ile Ile Leu Gly Ser Leu Tyr His
                580                 585                 590

Gly Asp His Leu Ser Arg Ala Met Tyr Gln Arg Ile Ser Asn Ile Glu
        595                 600                 605

Asp Leu Pro Pro Leu Tyr Thr Leu Asn Lys Pro Leu Leu Ser Gly Ile
    610                 615                 620

Ser Asn Ala Glu Ala Arg Gln Pro Gly Lys Ala Pro Asn Phe Ser Val
625                 630                 635                 640

Asn Trp Thr Val Gly Asp Ser Ala Ile Glu Val Ile Asn Ala Thr Thr
                645                 650                 655

Gly Lys Asp Glu Leu Gly Arg Ala Ser Arg Leu Cys Lys His Ala Leu
                660                 665                 670

Tyr Cys Arg Trp Met Arg Val His Gly Lys Val Pro Ser His Leu Leu
        675                 680                 685

Arg Ser Lys Ile Thr Lys Pro Asn Val Tyr His Glu Ser Lys Leu Ala
    690                 695                 700

Ala Lys Glu Tyr Gln Ala Ala Lys Ala Arg Leu Phe Thr Ala Phe Ile
705                 710                 715                 720

Lys Ala Gly Leu Gly Ala Trp Val Glu Lys Pro Thr Glu Gln Asp Gln
                725                 730                 735

Phe Ser Leu Thr Pro
                740

<210>  12
<211>  701
<212>  PRT
<213>  artificial sequence

<220>
<223>  ADAR2-isoform2

<400>  12

Met Asp Ile Glu Asp Glu Glu Asn Met Ser Ser Ser Ser Thr Asp Val
1               5                   10                  15

Lys Glu Asn Arg Asn Leu Asp Asn Val Ser Pro Lys Asp Gly Ser Thr
                20                  25                  30

Pro Gly Pro Gly Glu Gly Ser Gln Leu Ser Asn Gly Gly Gly Gly Gly

35                  40                  45

Pro Gly Arg Lys Arg Pro Leu Glu Glu Gly Ser Asn Gly His Ser Lys
    50                  55                  60

Tyr Arg Leu Lys Lys Arg Arg Lys Thr Pro Gly Pro Val Leu Pro Lys
65                  70                  75                  80

Asn Ala Leu Met Gln Leu Asn Glu Ile Lys Pro Gly Leu Gln Tyr Thr
                85                  90                  95

Leu Leu Ser Gln Thr Gly Pro Val His Ala Pro Leu Phe Val Met Ser
            100                 105                 110

Val Glu Val Asn Gly Gln Val Phe Glu Gly Ser Gly Pro Thr Lys Lys
        115                 120                 125

Lys Ala Lys Leu His Ala Ala Glu Lys Ala Leu Arg Ser Phe Val Gln
    130                 135                 140

Phe Pro Asn Ala Ser Glu Ala His Leu Ala Met Gly Arg Thr Leu Ser
145                 150                 155                 160

Val Asn Thr Asp Phe Thr Ser Asp Gln Ala Asp Phe Pro Asp Thr Leu
                165                 170                 175

Phe Asn Gly Phe Glu Thr Pro Asp Lys Ala Glu Pro Pro Phe Tyr Val
            180                 185                 190

Gly Ser Asn Gly Asp Asp Ser Phe Ser Ser Ser Gly Asp Leu Ser Leu
        195                 200                 205

Ser Ala Ser Pro Val Pro Ala Ser Leu Ala Gln Pro Pro Leu Pro Val
    210                 215                 220

Leu Pro Pro Phe Pro Pro Pro Ser Gly Lys Asn Pro Val Met Ile Leu
225                 230                 235                 240

Asn Glu Leu Arg Pro Gly Leu Lys Tyr Asp Phe Leu Ser Glu Ser Gly
                245                 250                 255

Glu Ser His Ala Lys Ser Phe Val Met Ser Val Val Val Asp Gly Gln
            260                 265                 270

Phe Phe Glu Gly Ser Gly Arg Asn Lys Lys Leu Ala Lys Ala Arg Ala
        275                 280                 285

Ala Gln Ser Ala Leu Ala Ala Ile Phe Asn Leu His Leu Asp Gln Thr
    290                 295                 300

Pro Ser Arg Gln Pro Ile Pro Ser Glu Gly Leu Gln Leu His Leu Pro
305                 310                 315                 320

Gln Val Leu Ala Asp Ala Val Ser Arg Leu Val Leu Gly Lys Phe Gly
                325                 330                 335

Asp Leu Thr Asp Asn Phe Ser Ser Pro His Ala Arg Arg Lys Val Leu
            340                 345                 350

Ala Gly Val Val Met Thr Thr Gly Thr Asp Val Lys Asp Ala Lys Val
        355                 360                 365

Ile Ser Val Ser Thr Gly Thr Lys Cys Ile Asn Gly Glu Tyr Met Ser
    370                 375                 380

Asp Arg Gly Leu Ala Leu Asn Asp Cys His Ala Glu Ile Ile Ser Arg
385                 390                 395                 400

Arg Ser Leu Leu Arg Phe Leu Tyr Thr Gln Leu Glu Leu Tyr Leu Asn
                405                 410                 415

Asn Lys Asp Asp Gln Lys Arg Ser Ile Phe Gln Lys Ser Glu Arg Gly
            420                 425                 430

Gly Phe Arg Leu Lys Glu Asn Val Gln Phe His Leu Tyr Ile Ser Thr
        435                 440                 445

Ser Pro Cys Gly Asp Ala Arg Ile Phe Ser Pro His Glu Pro Ile Leu
    450                 455                 460

Glu Glu Pro Ala Asp Arg His Pro Asn Arg Lys Ala Arg Gly Gln Leu
465                 470                 475                 480

Arg Thr Lys Ile Glu Ser Gly Glu Gly Thr Ile Pro Val Arg Ser Asn
                485                 490                 495

Ala Ser Ile Gln Thr Trp Asp Gly Val Leu Gln Gly Glu Arg Leu Leu
            500                 505                 510

Thr Met Ser Cys Ser Asp Lys Ile Ala Arg Trp Asn Val Val Gly Ile
        515                 520                 525

Gln Gly Ser Leu Leu Ser Ile Phe Val Glu Pro Ile Tyr Phe Ser Ser
    530                 535                 540

Ile Ile Leu Gly Ser Leu Tyr His Gly Asp His Leu Ser Arg Ala Met
545                 550                 555                 560

Tyr Gln Arg Ile Ser Asn Ile Glu Asp Leu Pro Pro Leu Tyr Thr Leu
                565                 570                 575

Asn Lys Pro Leu Leu Ser Gly Ile Ser Asn Ala Glu Ala Arg Gln Pro
            580                 585                 590

Gly Lys Ala Pro Asn Phe Ser Val Asn Trp Thr Val Gly Asp Ser Ala
        595                 600                 605

Ile Glu Val Ile Asn Ala Thr Thr Gly Lys Asp Glu Leu Gly Arg Ala
    610                 615                 620

Ser Arg Leu Cys Lys His Ala Leu Tyr Cys Arg Trp Met Arg Val His
625                 630                 635                 640

Gly Lys Val Pro Ser His Leu Leu Arg Ser Lys Ile Thr Lys Pro Asn
                645                 650                 655

Val Tyr His Glu Ser Lys Leu Ala Ala Lys Glu Tyr Gln Ala Ala Lys
            660                 665                 670

Ala Arg Leu Phe Thr Ala Phe Ile Lys Ala Gly Leu Gly Ala Trp Val
        675                 680                 685

Glu Lys Pro Thr Glu Gln Asp Gln Phe Ser Leu Thr Pro
    690                 695                 700

<210>  13
<211>  7
<212>  PRT
<213>  artificial sequence

<220>
<223>  nuclear localization signal sequence

<400>  13

Pro Lys Lys Lys Arg Lys Val
1               5

<210>  14
<211>  36
<212>  PRT
<213>  artificial sequence

<220>
<223>  mitochondrial localization signal sequence

<400>  14

Met Leu Phe Asn Leu Arg Ile Leu Leu Asn Asn Ala Ala Phe Arg Asn
1               5                   10                  15

Gly His Asn Phe Met Val Arg Asn Phe Arg Cys Gly Gln Pro Leu Gln
            20                  25                  30

Asn Lys Val Gln
        35

## Description

### Technical field

The present invention belongs to the field of biology. Specifically, the present invention relates to the use of artificially constructed RNA editing enzymes for RNA-directed editing and related applications.

### Background

DNA is the most important genetic material in organisms. At present, a large part of the known human diseases are caused by genetic mutations, and single-base mutations are the largest category. Therefore, the development of a method to change the sequence of a single base in the genome and repair the pathogenic single base mutations efficiently and accurately is of great significance for the research and treatment of genetic diseases.

The current gene therapy strategies for single-base mutation diseases are mainly to treat diseases by directly repairing or replacing mutant genes at the DNA or RNA level. The main methods are the base editing systems ABE and CBE for DNA based on CRISPR technology. These technologies can perform base editing to a certain extent, but there are still many shortcomings:
(1) The current CRISPR-mediated base editing system has insufficient precision and low efficiency for single-base editing. There is a common editing window, and additional mutations will be introduced when editing the target site.
(2) The CRISPR system is very large. The current gene transfer technology is difficult to package the entire CRISPR system in the same system for delivery at one time. Therefore, there is inefficient gene transfer during the treatment process and the editing efficiency is low.
(3) Limited by the size of the transgene, some genomic loci are difficult to transfer.
(4) Immune response and toxicity caused by the Cas protein as a bacterial protein.
(5) Mutations during gene insertion or unexpected events during integration.

It is especially important that the long-term safety concerns of genomic DNA editing have always been a big problem because the changes in genomic DNA will accompany the cell for a lifetime.

Therefore, those skilled in the art urgently need to develop a method that can effectively edit genes in order to perform effective gene therapy on single-base mutation diseases.

### Summary of the invention

The purpose of the present invention is to provide a method and application that can effectively edit genes.

In a first aspect of the present invention, it provides an RNA editing enzyme, comprising:
(a) a RNA recognition domain, the RNA recognition domain is used to recognize the RNA recognition sequence of the RNA sequence to be edited, and bind to the RNA recognition sequence;
(b) a utility domain, which is used for nucleotide editing of the RNA sequence to be edited;
wherein, the RNA recognition domain and the utility binding domain are operably linked.

In another preferred embodiment, the utility domain is selected from the group consisting of the deamination catalytic domain of ADAR family members, the deamination catalytic domain of APOBEC family members, RNA methylase, RNA demethylase, added uracil synthase, and a combination thereof.

In another preferred embodiment, the RNA recognition domain contains n recognition units, and each recognition unit is used to recognize an RNA base, wherein n is a positive integer of 5-30.

In another preferred example, n is 6-24, more preferably 8-20, for example, 6, 7, 8, 9, 10, 11, 12, 13, 14, 15, 16, 17, 18, 19 , 20, 21, 22, 23, 24.

In another preferred example, each recognition unit is an α-helix repeated sequence, and three amino acids at a specific position on the repeated sequence are responsible for binding RNA bases.

In another preferred example, the n recognition units are connected in series to form an RNA base recognition region.

In another preferred example, the RNA recognition domain further includes an optional protection region located on both sides of the RNA base recognition region to protect the RNA base recognition region.

In another preferred example, the RNA recognition domain is derived from PUF protein.

In another preferred example, the RNA recognition domain does not include the domain upstream of the RNA base recognition region in the PUF protein (except the protection region), but may or may not include the domain downstream of the RNA base recognition region in the PUF protein (except the protection region).

In another preferred example, the protection region is a non-functional repeated sequence at both ends of the RNA base recognition region.

In another preferred example, the RNA editing enzyme further includes one or more elements selected from the group consisting of linker peptide, tag sequence, signal peptide sequence, location peptide sequence, and a combination thereof.

In another preferred embodiment, the location peptide sequence includes a nuclear localization signal sequence, a mitochondrial localization signal sequence, and a combination thereof.

In another preferred example, the one or more elements are operably linked to the RNA recognition domain and utility domain.

In another preferred embodiment, the one or more elements are independently located at the N-terminal and/or C-terminal and/or middle of the RNA editing enzyme.

In another preferred embodiment, the signal peptide is located at the N-terminus of the RNA editing enzyme.

In another preferred example, the linker peptide, tag sequence, and/or location peptide signal is each independently located between the recognition domain and the utility domain.

In another preferred embodiment, the RNA is single-stranded.

In another preferred example, the RNA editing enzyme includes an RNA recognition domain and a utility domain, as well as an optional linker peptide, tag sequence, signal peptide sequence and/or location peptide sequence.

In another preferred example, the structure of the RNA editing enzyme is shown in any one of the following Formula I to formula IV:

D-L2-A-L1-B (I);

D-L2-B-L1-A (II);

A-L1-B-L2-D (III);

B-L1-A-L2-D (IV);

wherein each "-" is independently a linker peptide or a peptide bond;
A is a RNA recognition domain;
B is a utility domain;
L1 and L2 is each independently none or a linker peptide;
D is none or a location peptide.

In another preferred embodiment, the location peptide is located at the N-terminal, C-terminal or middle of the RNA editing enzyme.

In another preferred embodiment, the RNA recognition domain is a domain capable of recognizing and binding RNA, preferably from an RNA binding protein.

In another preferred embodiment, the RNA recognition domain is a PUF protein fragment.

In another preferred embodiment, the PUF protein fragment is a fragment of the PUF protein that can recognize the RNA-binding domain.

In another preferred embodiment, the PUF protein includes a PUF protein and a homologous protein thereof.

In another preferred embodiment, the PUF protein is derived from a mammal, preferably from a primate, and more preferably from a human.

In another preferred embodiment, the RNA recognition domain has an amino acid sequence as shown in SEQ ID NO.: 1.

GRSRLLEDFRNNRYPNLQLREIAGHIMEFSQDQHGSRFIQLKLERATPAERQ
LVFNEILQAAYQLMVDVFGNYVIQKFFEFGSLEQKLALAERIRGHVLSLALQMY
GCRVIQKALEFIPSDQQNEMVRELDGHVLKCVKDQNGNHVVQKCIECVQPQSL
QFIIDAFKGQVFALSTHPYGCRVIQRILEHCLPDQTLPILEELHQHTEQLVQDQY
GNYVIQHVLEHGRPEDKSKIVAEIRGNVLVLSQHKFASNVVEKCVTHASRTERA

VLIDEVCTMNDGPHSALYTMMKDQYANYVVQKMIDVAEPGQRKIVMHKIRPH
IATLRKYTYGKHILAKLEKYYMKNGVDLG (SEQ ID NO.: 1)

In another preferred embodiment, the ADAR family member includes: dADAR, ADAR1, ADAR2, TadA, and a combination thereof.

In another preferred embodiment, the member of the ADAR family is derived from a human or Drosophila or bacteria.

In another preferred embodiment, the ADAR family member includes: Drosophila ADAR, human ADAR1, human ADAR2, E. coli TadA, or a combination thereof.

In another preferred embodiment, the ADAR1 includes a natural ADAR1 and ADAR1 mutant.

In another preferred example, the ADAR1 mutant has a mutation at position 1008 in the amino acid sequence corresponding to the natural ADAR1; preferably, the glutamic acid (E) at position 1008 is mutated to glutamine (Q) .

In another preferred example, the effector domain is derived from ADAR1 and has the amino acid sequence as shown in SEQ ID NO.: 2: 
KAERMGFTEVTPVTGASLRRTMLLLSRSPEAQPKTLPLTGSTFHDQIAMLS
HRCFNTLTNSFQPSLLGRKILAAIIMKKDSEDMGVVVSLGTGNRCVKGDSLSLK
GETVNDCHAEIISRRGFIRFLYSELMKYNSQTAKDSIFEPAKGGEKLQIKKTVSF
HLYISTAPCGDGALFDKSCSDRAMESTESRHYPVFENPKQGKLRTKVENGEGTI
PVESSDIVPTWDGIRLGERLRTMSCSDKILRWNVLGLQGALLTHFLQPIYLKSVT
LGYLFSQGHLTRAICCRVTRDGSAFEDGLRHPFIVNHPKVGRVSIYDSKRQSGK
TKETSVNWCLADGYDLEILDGTRGTVDGPRNELSRVSKKNIFLLFKKLCSFRYR
RDLLRLSYGEAKKAARDYETAKNYFKKGLKDMGYGNWISKPQEEKNFYLCPV
(SEQ ID NO.: 2)
 or the effector domain is the ADAR1-E1008Q effector domain, and its amino acid sequence is the same as SEQ ID No.: 2, but the position 211 in SEQ ID No.: 2 is mutated from E to Q: 
KAERMGFTEVTPVTGASLRRTMLLLSRSPEAQPKTLPLTGSTFHDQIAMLS
HRCFNTLTNSFQPSLLGRKILAAIIMKKDSEDMGVVVSLGTGNRCVKGDSLSLK
GETVNDCHAEIISRRGFIRFLYSELMKYNSQTAKDSIFEPAKGGEKLQIKKTVSF
HLYISTAPCGDGALFDKSCSDRAMESTESRHYPVFENPKQGKLRTKVENGQGTI
PVESSDIVPTWDGIRLGERLRTMSCSDKILRWNVLGLQGALLTHFLQPIYLKSVT
LGYLFSQGHLTRAICCRVTRDGSAFEDGLRHPFIVNHPKVGRVSIYDSKRQSGK
TKETSVNWCLADGYDLEILDGTRGTVDGPRNELSRVSKKNIFLLFKKLCSFRYR
RDLLRLSYGEAKKAARDYETAKNYFKKGLKDMGYGNWISKPQEEKNFYLCPV
 (SEQ ID No.: 2, among them, the position 211 is mutated from E to Q).

In another preferred embodiment, the ADAR2 mutant is mutated at position 488 in the amino acid sequence corresponding to the natural ADAR2; preferably, the glutamate (E) at position 488 is mutated to glutamine (Q) .

In another preferred example, the effector domain has the amino acid sequence as shown in SEQ ID NO.: 2, 3, or 4 or its derivative sequence.

In another preferred example, the derivative sequence includes: SEQ ID No.: 2, and the position 211 is mutated from E to Q); SEQ ID No.: 3, wherein the position 227 is mutated from E to Q; SEQ ID No.: 4, wherein the position 187 is mutated from E to Q.

In another preferred embodiment, the effector domain is selected from the group consisting of:
(i) an ADAR2-isoform1 effector domain with the amino acid sequence as shown in SEQ ID NO.: 3
(ii) an ADAR2-isoform1-E488Q effector domain having the amino acid sequence as shown in SEQ ID NO.: 3 and mutated from E to Q at position 227; and
(iii) an ADAR2-isoform2 effector domain having the amino acid sequence as shown in SEQ ID NO.: 4;
(iv) an ADAR2-isoform2-E488Q effector domain having the amino acid sequence as shown in SEQ ID NO.: 4 and mutated from E to Q at position 187;

In another preferred example, the amino acid sequence of the effector domain of ADAR2-isoform1 is shown as follows:

DQTPSRQPIPSEGLQLHLPQVLADAVSRLVLGKFGDLTDNFSSPHARRKVL
AGVVMTTGTDVKDAKVISVSTGTKCINGEYMSDRGLALNDCHAEIISRRSLLRF
LYTQLELYLNNKDDQKRSIFQKSERGGFRLKENVQFHLYISTSPCGDARIFSPHE
PILEGSRSYTQAGVQWCNHGSLQPRPPGLLSDPSTSTFQGAGTTEPADRHPNRK
ARGQLRTKIESGEGTIPVRSNASIQTWDGVLQGERLLTMSCSDKIARWNVVGIQ
GSLLSIFVEPIYFSSIILGSLYHGDHLSRAMYQRISNIEDLPPLYTLNKPLLSGISNA
EARQPGKAPNFSVNWTVGDSAIEVINATTGKDELGRASRLCKHALYCRWMRV
HGKVPSHLLRSKITKPNVYHESKLAAKEYQAAKARLFTAFIKAGLGAWVEKPT
EQDQFSLTP (SEQ ID NO.: 3)

In another preferred example, the amino acid sequence of the ADAR2-isoform1-E488Q effector domain is shown as follows:

DQTPSRQPIPSEGLQLHLPQVLADAVSRLVLGKFGDLTDNFSSPHARRKVL
AGVVMTTGTDVKDAKVISVSTGTKCINGEYMSDRGLALNDCHAEIISRRSLLRF
LYTQLELYLNNKDDQKRSIFQKSERGGFRLKENVQFHLYISTSPCGDARIFSPHE
PILEGSRSYTQAGVQWCNHGSLQPRPPGLLSDPSTSTFQGAGTTEPADRHPNRK
ARGQLRTKIESGQGTIPVRSNASIQTWDGVLQGERLLTMSCSDKIARWNVVGIQ
GSLLSIFVEPIYFSSIILGSLYHGDHLSRAMYQRISNIEDLPPLYTLNKPLLSGISNA

EARQPGKAPNFSVNWTVGDSAIEVINATTGKDELGRASRLCKHALYCRWMRV
HGKVPSHLLRSKITKPNVYHESKLAAKEYQAAKARLFTAFIKAGLGAWVEKPT
EQDQFSLTP (the yellow amino acid is the position 488 amino acid mutation site)
(SEQ ID No.: 3, wherein the position 227 is mutated from E to Q)

In another preferred example, the amino acid sequence of the ADAR2-isoform2 effector domain is as follows:

DQTPSRQPIPSEGLQLHLPQVLADAVSRLVLGKFGDLTDNFSSPHARRKVL
AGVVMTTGTDVKDAKVISVSTGTKCINGEYMSDRGLALNDCHAEIISRRSLLRF
LYTQLELYLNNKDDQKRSIFQKSERGGFRLKENVQFHLYISTSPCGDARIFSPHE
PILEEPADRHPNRKARGQLRTKIESGEGTIPVRSNASIQTWDGVLQGERLLTMSC
SDKIARWNVVGIQGSLLSIFVEPIYFSSIILGSLYHGDHLSRAMYQRISNIEDLPPL
YTLNKPLLSGISNAEARQPGKAPNFSVNWTVGDSAIEVINATTGKDELGRASRL
CKHALYCRWMRVHGKVPSHLLRSKITKPNVYHESKLAAKEYQAAKARLFTAFI
KAGLGAWVEKPTEQDQFSLTP(SEQ ID NO.: 4)

In another preferred example, the amino acid sequence of the ADAR2-isoform2-E488Q effector domain is as follows:
DQTPSRQPIPSEGLQLHLPQVLADAVSRLVLGKFGDLTDNFSSPHARRKVL AGVVMTTGTDVKDAKVISVSTGTKCINGEYMSDRGLALNDCHAEIISRRSLLRF LYTQLELYLNNKDDQKRSIFQKSERGGFRLKENVQFHLYISTSPCGDARIFSPHE PILEEPADRHPNRKARGQLRTKIESGQGTIPVRSNASIQTWDGVLQGERLLTMSC SDKIARWNVVGIQGSLLSIFVEPIYFSSIILGSLYHGDHLSRAMYQRISNIEDLPPL YTLNKPLLSGISNAEARQPGKAPNFSVNWTVGDSAIEVINATTGKDELGRASRL CKHALYCRWMRVHGKVPSHLLRSKITKPNVYHESKLAAKEYQAAKARLFTAFI KAGLGAWVEKPTEQDQFSLTP (yellow amino acid is the position 488 amino acid mutation site) (SEQ ID No.: 4, the position 187 is mutated from E to Q)

In another preferred embodiment, the APOBEC family member includes: Apobecl, Apobec3A, Apobec3G, or a combination thereof.

In another preferred embodiment, the APOBEC family member is derived from a human or mouse (rat), preferably from a human.

In another preferred embodiment, the APOBEC family member is selected from the group consisting of human Apobecl, human Apobec3A, human Apobec3B, human Apobec3C, human Apobec3D, human Apobec3F, human Apobec3G, human Apobec3H, human AID, mouse Apobecl, mouse Apobec3A, mouse AID, rat Apobecl, rat Apobec3A, rat AID, and a combination thereof.

In another preferred example, the APOBEC3A has the amino acid sequence as shown in SEQ ID NO.: 5 (full-length amino acid sequence). 
MEASPASGPRHLMDPHIFTSNFNNGIGRHKTYLCYEVERLDNGTSVKMDQ
HRGFLHNQAKNLLCGFYGRHAELRFLDLVPSLQLDPAQIYRVTWFISWSPCFSW
GCAGEVRAFLQENTHVRLRIFAARIYDYDPLYKEALQMLRDAGAQVSIMTYDE
FKHCWDTFVDHQGCPFQPWDGLDEHSQALSGRLRAILQNQGN (SEQ ID NO.:
 5).

In another preferred embodiment, the RNA methylase includes: METTL3, METTL14, and a combination thereof.

In another preferred embodiment, the RNA demethylase is Alpha-ketoglutarate-dependent dioxygenase FTO.

In another preferred embodiment, the added uracil synthase is pseudoouridine7.

In another preferred example, the editing window of the RNA editing enzyme is position 7-14, preferably position 8-13, and more preferably position 9-11, wherein the calculation starts from the first position of the 5' end of the PUF binding site (that is, the first position).

In another preferred embodiment, the RNA editing enzyme does not contain RNA and/or DNA.

In another preferred embodiment, the RNA recognition domain and the utility domain in the RNA editing enzyme are connected in a head-to-tail, head-to-head, tail-to-head, or tail-to-tail manner.

In another preferred embodiment, the RNA recognition domain and the utility domain in the RNA editing enzyme are connected directly or through a linker peptide.

In another preferred example, the C-terminal or N-terminal of the RNA editing enzyme further includes an NLS sequence and an MLS sequence.

NLS nuclear localization signal sequence: PKKKRKV (SEQ ID No.: 13).

MLS mitochondrial localization signal sequence: MLFNLRILLNNAAFRNGHNFMVRNFRCGQPLQNKVQ (SEQ ID No.: 14).

In another preferred embodiment, the linker peptide is a none or a flexible peptide.

In another preferred embodiment, the linker peptide is selected from the group consisting of Linker2, Linker7, XTEN, Linker20, Linker40, and a combination thereof.

In another preferred example, the length of the linker peptide is 0-40 aa, preferably 2-20 aa.

In another preferred example, the linker peptide has an amino acid sequence as shown in any one of SEQ ID NO.: 6-9:
Linker2: EF
Linker7: EFTGNGS(SEQ ID NO.: 6)
XTEN: SGSETPGTSESATPES (SEQ ID NO.: 7)
Linker20: DQTPSRQPIPSEGLQLHLPQ (SEQ ID NO.: 8)
Linker41: KAERMGFTEVTPVTGASLRRTMLLLSRSPEAQPKTLPLTGS (SEQ ID NO.: 9)

In a second aspect of the present invention, it provides an isolated polynucleotide which encodes the RNA editing enzyme according to the first aspect of the present invention.

In a third aspect of the present invention, it provides a vector, which comprises the polynucleotide according to the second aspect of the present invention.

In another preferred embodiment, the vector includes DNA and RNA.

In another preferred embodiment, the vector is selected from the group consisting of: plasmid, viral vector, transposon, and a combination thereof.

In another preferred embodiment, the vector includes DNA virus and retroviral vector.

In another preferred embodiment, the vector is selected from the group consisting of a lentiviral vector, an adenovirus vector, an adeno-associated virus vector, and a combination thereof.

In another preferred embodiment, the vector is a lentiviral vector.

In another preferred embodiment, the vector includes one or more promoters, which are operably linked to the nucleic acid sequence, enhancer, intron, transcription termination signal, polyadenylation sequence, origin of replication, selected marker, nucleic acid restriction site, and/or homologous recombination site.

In another preferred example, the vector is a vector containing or inserted with the polynucleotide of the second aspect of the present invention.

In a fourth aspect of the present invention, it provides a host cell, which contains the vector according to the third aspect of the present invention, or the exogenous polynucleotide according to the second aspect of the present invention integrated into the chromosome, or express the RNA editing enzyme according to the first aspect of the present invention.

In another preferred embodiment, the host cell is a prokaryotic cell or a eukaryotic cell.

In another preferred embodiment, the host cell is a human cell or a non-human mammalian cell.

In a fifth aspect of the present invention, it provides a preparation, wherein the preparation comprises the RNA editing enzyme according to the first aspect of the present invention, or the polynucleotide according to the second aspect, or the vector according to the third aspect, and a pharmaceutically acceptable carrier or excipient.

In another preferred embodiment, the preparation is a liquid preparation.

In a sixth aspect of the present invention, it provides a use of the RNA editing enzyme according to the first aspect, or the polynucleotide according to the second aspect, or the host cell according to the fourth aspect of the present invention for the preparation of
(a) a drug or preparation for gene therapy; and/or
(b) a reagent for editing RNA.

In another preferred example, the editing RNA includes mutating A to G and/or mutating C to U in RNA.

In a seventh aspect of the present invention, it provides a method for editing RNA, the method comprising the steps:
(1) providing RNA to be edited and the RNA editing enzyme according to the first aspect of the present invention; and
(2) using the RNA editing enzyme of claim 1 to edit the RNA.

In another preferred embodiment, the method is in vitro or in vivo.

In another preferred embodiment, the method is for non-diagnostic and non-therapeutic purposes.

In another preferred example, the editing RNA includes mutating A to G and/or mutating C to U in RNA.

In a eighth aspect of the present invention, it provides a method for preparing the RNA editing enzyme of the first aspect of the present invention, the method comprising the steps:
under a suitable expression condition, culturing the host cell according to the fourth aspect of the present invention, thereby expressing the RNA editing enzyme; and isolating the RNA editing enzyme.

In another preferred embodiment, the host cell is a prokaryotic cell or a eukaryotic cell.

In a ninth aspect of the present invention, it provides a method for treating diseases, the method comprising: administering the RNA editing enzyme according to the first aspect of the present invention or the polynucleotide according to the second aspect of the present invention or the vector according to the third aspect of the present invention, or the preparation of the fifth aspect of the present invention to a subject in need.

It should be understood that, within the scope of the present invention, each technical feature of the present invention described above and in the following (as examples) may be combined with each other to form a new or preferred technical solution, which is not listed here due to space limitations.

### Description of Figure

Figure 1 shows the construction of different A→G RNA editing enzymes.
Figure 2 shows the use of PARSEs to edit target RNA.
Figure 3 shows that the enzyme produced by replacing the positions of PUF and ADAR cannot edit the target RNA.
Figure 4 shows the efficiency and off-target rate analysis of PARSEs on target RNA editing.
Figure 5 shows the use of ePARSE1 to repair the abnormal RNA editing event of the GRIA2 gene.
Figure 6 shows the use of ePARSE2 to repair disease-causing point mutations in related genes.
Figure 7 shows that extending the PUF recognition domain in the PARSE system can significantly improve the RNA editing accuracy of the PARSE system.
Figure 8 shows the construction of APRSEs to edit target RNA.
Figure 9 shows the use of APRSE to repair disease-causing point mutations in related genes.
Figure 10 shows that extending the PUF recognition domain in the APRSE system can significantly improve the RNA editing accuracy of the APRSE system.
Figure 11 shows a schematic diagram of the structure of the PUF element.

### Detailed Description

After extensive and in-depth research, the inventors have developed an RNA editing enzyme with a unique structure for the first time. The inventors have unexpectedly discovered that a novel RNA editing enzyme based on the RNA-binding recognition domain and utility domain can very effectively target specific RNA regions and perform efficient and accurate RNA editing. The RNA editing enzyme of the present invention can not only perform RNA editing efficiently and accurately, but also can effectively prevent back mutation, so that it has the advantages of more flexibility, safety, efficiency and the like. On this basis, the inventors have completed the present invention.

### Term Description

Unless otherwise defined, all technical and scientific terms used herein have the same meaning as commonly understood by those of ordinary skill in the art to which the present invention belongs.

As used herein, when used in reference to a specifically recited value, the term "about" means that the value can vary from the recited value by no more than 1%. For example, as used herein, the expression "about 100" includes all values between 99 and 101 (e.g., 99.1, 99.2, 99.3, 99.4, etc.).

As used herein, the term "containing" or "comprising (including)" can be open, semi-closed, and closed. In other words, the term also includes "substantially consisting of" or "consisting of".

### PUF protein (Pumilio homolog 1)

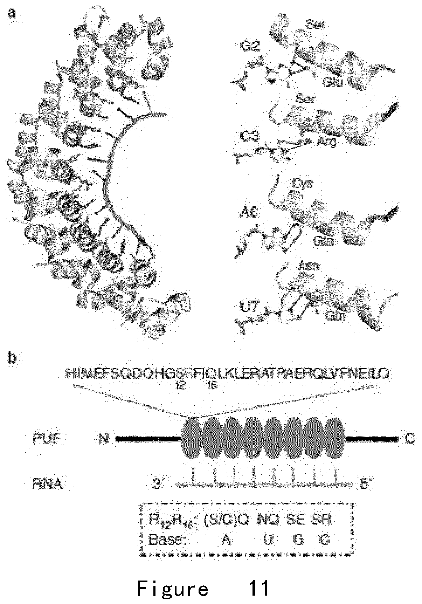

PUF protein is a sequence-specific RNA binding protein with a conserved RNA binding domain, which regulates the stability or translation efficiency of mRNA by binding to the 3'-UTR of the target mRNA. A typical PUF binding domain contains 8 α-helix repeated sequence, each repeated sequence is 36 amino acids, responsible for identifying and binding 1 base, at both ends of the binding domain, each has a non-functional repeated sequence to protect the 8 repeated sequences in the middle. Three amino acids at specific positions on the repeated sequence of α-helix are responsible for binding RNA bases and wherein the side chain amino acids at positions 12 and 16 bind to RNA bases through hydrogen bonds, and the amino acid at position 13 serves as an auxiliary binding. Different combinations of amino acids are responsible for recognizing different bases (see Figure 11). After modification and design of each repeated sequence of the PUF protein, the newly designed PUF of this application can recognize and bind any 8-base RNA sequence. After modification, the number of PUF repeated sequences can be increased and decreased to expand the PUF's ability to bind RNA, so that the PUF of the present application can recognize 6 to 16 bases, and further can recognize 20 bases. In different species, PUF protein has many homologous proteins, which have similar sequence characteristics and similar functions to PUF protein.

In a preferred example of the present invention, human-derived PUM1 is selected as a tool for RNA recognition.

### ADAR

ADAR (Double-stranded RNA-specific adenosine deaminase) protein is a type of RNA deaminase that acts on double-stranded RNA. It catalyzes the hydrolysis and deamination of adenosine in double-stranded RNA to form inosine (adenosine-to -inosine, A-to-I), known as A-to-I RNA editing, inosine (I) is recognized as guanosine (G) during translation, realizing A-to-G RNA editing. The main members of the protein family are ADAR1, ADAR2 and ADAR3. The gene editing produced by this enzyme will affect the expression and function of genes, including by changing the mRNA translation of codons, thereby changing the amino acid sequence of the protein; by changing the splice site recognition sequence, thereby performing pre-mRNA splicing; achieving RNA stability by changing the sequence involved in nuclease recognition; RNA viral genome changes sequence during viral RNA replication to achieve genetic stability and regulate some structure-dependent RNA metabolic activities, such as microRNA production, targeting or protein-RNA interaction.

Representative ADARs include ADAR1, ADAR2-isoform1 and ADAR2-isoform1 or the homologous proteins thereof.

A representative full-length amino acid sequence of ADAR1 is as follows:

MNPRQGYSLSGYYTHPFQGYEHRQLRYQQPGPGSSPSSFLLKQIEFLKGQL
PEAPVIGKQTPSLPPSLPGLRPRFPVLLASSTRGRQVDIRGVPRGVHLRSQGLQR
GFQHPSPRGRSLPQRGVDCLSSHFQELSIYQDQEQRILKFLEELGEGKATTAHDL
SGKLGTPKKEINRVLYSLAKKGKLQKEAGTPPLWKIAVSTQAWNQHSGVVRPD
GHSQGAPNSDPSLEPEDRNSTSVSEDLLEPFIAVSAQAWNQHSGVVRPDSHSQG
SPNSDPGLEPEDSNSTSALEDPLEFLDMAEIKEKICDYLFNVSDSSALNLAKNIGL
TKARDINAVLIDMERQGDVYRQGTTPPIWHLTDKKRERMQIKRNTNSVPETAP
AAIPETKRNAEFLTCNIPTSNASNNMVTTEKVENGQEPVIKLENRQEARPEPARL
KPPVHYNGPSKAGYVDFENGQWATDDIPDDLNSIRAAPGEFRAIMEMPSFYSH
GLPRCSPYKKLTECQLKNPISGLLEYAQFASQTCEFNMIEQSGPPHEPRFKFQVVI
NGREFPPAEAGSKKVAKQDAAMKAMTILLEEAKAKDSGKSEESSHYSTEKESE
KTAESQTPTPSATSFFSGKSPVTTLLECMHKLGNSCEFRLLSKEGPAHEPKFQYC
VAVGAQTFPSVSAPSKKVAKQMAAEEAMKALHGEATNSMASDNQPEGMISES
LDNLESMMPNKVRKIGELVRYLNTNPVGGLLEYARSHGFAAEFKLVDQSGPPH
EPKFVYQAKVGGRWFPAVCAHSKKQGKQEAADAALRVLIGENEKAERMGFTE
VTPVTGASLRRTMLLLSRSPEAQPKTLPLTGSTFHDQIAMLSHRCFNTLTNSFQP
SLLGRKILAAIIMKKDSEDMGVVVSLGTGNRCVKGDSLSLKGETVNDCHAEIIS
RRGFIRFLYSELMKYNSQTAKDSIFEPAKGGEKLQIKKTVSFHLYISTAPCGDGA
LFDKSCSDRAMESTESRHYPVFENPKQGKLRTKVENGEGTIPVESSDIVPTWDGI
RLGERLRTMSCSDKILRWNVLGLQGALLTHFLQPIYLKSVTLGYLFSQGHLTRA
ICCRVTRDGSAFEDGLRHPFIVNHPKVGRVSIYDSKRQSGKTKETSVNWCLADG
YDLEILDGTRGTVDGPRNELSRVSKKNIFLLFKKLCSFRYRRDLLRLSYGEAKK
AARDYETAKNYFKKGLKDMGYGNWISKPQEEKNFYLCPV* (SEQ ID No.: 10)

A representative full-length amino acid sequence of ADAR2-isoform1 is as follows:

MDIEDEENMSSSSTDVKENRNLDNVSPKDGSTPGPGEGSQLSNGGGGGPG
RKRPLEEGSNGHSKYRLKKRRKTPGPVLPKNALMQLNEIKPGLQYTLLSQTGPV
HAPLFVMSVEVNGQVFEGSGPTKKKAKLHAAEKALRSFVQFPNASEAHLAMG
RTLSVNTDFTSDQADFPDTLFNGFETPDKAEPPFYVGSNGDDSFSSSGDLSLSAS
PVPASLAQPPLPVLPPFPPPSGKNPVMILNELRPGLKYDFLSESGESHAKSFVMS
VVVDGQFFEGSGRNKKLAKARAAQSALAAIFNLHLDQTPSRQPIPSEGLQLHLP
QVLADAVSRLVLGKFGDLTDNFSSPHARRKVLAGVVMTTGTDVKDAKVISVST
GTKCINGEYMSDRGLALNDCHAEIISRRSLLRFLYTQLELYLNNKDDQKRSIFQK
SERGGFRLKENVQFHLYISTSPCGDARIFSPHEPILEGSRSYTQAGVQWCNHGSL
QPRPPGLLSDPSTSTFQGAGTTEPADRHPNRKARGQLRTKIESGEGTIPVRSNASI
QTWDGVLQGERLLTMSCSDKIARWNVVGIQGSLLSIFVEPIYFSSIILGSLYHGD
HLSRAMYQRISNIEDLPPLYTLNKPLLSGISNAEARQPGKAPNFSVNWTVGDSAI
EVINATTGKDELGRASRLCKHALYCRWMRVHGKVPSHLLRSKITKPNVYHESK
LAAKEYQAAKARLFTAFIKAGLGAWVEKPTEQDQFSLTP*(SEQ ID No.:11)

A representative full-length amino acid sequence of ADAR2-isoform2 is as follows:

MDIEDEENMSSSSTDVKENRNLDNVSPKDGSTPGPGEGSQLSNGGGGGPG
RKRPLEEGSNGHSKYRLKKRRKTPGPVLPKNALMQLNEIKPGLQYTLLSQTGPV
HAPLFVMSVEVNGQVFEGSGPTKKKAKLHAAEKALRSFVQFPNASEAHLAMG
RTLSVNTDFTSDQADFPDTLFNGFETPDKAEPPFYVGSNGDDSFSSSGDLSLSAS
PVPASLAQPPLPVLPPFPPPSGKNPVMILNELRPGLKYDFLSESGESHAKSFVMS

VVVDGQFFEGSGRNKKLAKARAAQSALAAIFNLHLDQTPSRQPIPSEGLQLHLP
QVLADAVSRLVLGKFGDLTDNFSSPHARRKVLAGVVMTTGTDVKDAKVISVST
GTKCINGEYMSDRGLALNDCHAEIISRRSLLRFLYTQLELYLNNKDDQKRSIFQK
SERGGFRLKENVQFHLYISTSPCGDARIFSPHEPILEEPADRHPNRKARGQLRTKI
ESGEGTIPVRSNASIQTWDGVLQGERLLTMSCSDKIARWNVVGIQGSLLSIFVEPI
YFSSIILGSLYHGDHLSRAMYQRISNIEDLPPLYTLNKPLLSGISNAEARQPGKAP
NFSVNWTVGDSAIEVINATTGKDELGRASRLCKHALYCRWMRVHGKVPSHLL
RSKITKPNVYHESKLAAKEYQAAKARLFTAFIKAGLGAWVEKPTEQDQFSLTP*
(SEQ ID No.: 12)

### APOBECs protein

APOBEC (apolipoprotein B mRNA editing enzyme, catalytic polypeptide-like) is an evolutionary conserved cytidine deaminase, which acts on the single-stranded region of DNA/RNA and catalyzes the hydrolysis and deamination of cytidine on single-stranded DNA/RNA to form uridine (cytosine-to-uracil, C-to-U), realizing C-to-U DNA/RNA editing. The main members of this protein family are AID, Apobecl, Apobec2, Apobec3A, Apobec3B, Apobec3C, Apobec3D, Apobec3F, Apobec3G, Apobec3H and Apobec4.

### RNA editing enzyme

As used herein, "RNA editing enzyme of the present invention", "artificial RNA editing enzyme", "fusion protein" or "polypeptide of the present invention" all refer to the RNA editing enzyme described in the first aspect of the present invention.

The RNA editing enzyme of the present invention includes:
(a) a RNA recognition domain, the RNA recognition domain is used to recognize the RNA recognition sequence of the RNA sequence to be edited, and bind to the RNA recognition sequence;
(b) a utility domain, which is used for nucleotide editing of the RNA sequence to be edited;
wherein, the RNA recognition domain and the utility binding domain are operably connected.

As used herein, "operable (operably) connected (to)" or "operable (operably) linked (to)" refers to a parallel relationship in which the elements are in a relationship that allows them to function as expected. For example, if the RNA recognition domain and the utility domain are connected (directly connected, or connected through a connecting element, or connected through other functional elements located between the two), then as long as the RNA recognition domain and utility domain can perform their respective functions, that is, the RNA recognition domain recognizes and binds to a predetermined RNA recognition sequence, and the utility domain can perform nucleotide editing on the RNA sequence to be edited, and the two are operatively connected. Similarly, if a promoter can cause transcription or expression of a coding sequence, the promoter is operably linked to the coding sequence.

As used herein, the term "RNA editing enzyme of the present invention" also includes variant forms of the sequence having the above-mentioned activity. These variant forms include (but are not limited to): 1-3 (usually 1-2, more preferably 1) amino acid deletions, insertions and/or substitutions, and adding or deleting one or several (usually within 3, preferably within 2, more preferably within 1) amino acid at the C-terminal and/or N-terminal. For example, in this field, substitution with close or similar amino acids usually does not change the function of the protein. For another example, adding or deleting one or several amino acids at the C-terminus and/or N-terminus usually does not change the structure and function of the protein. In addition, the term also includes the polypeptide of the present invention in monomeric and multimeric forms. The term also includes linear and non-linear polypeptides (such as cyclic peptides).

The present invention also includes active fragments, derivatives and analogs of the above RNA editing enzymes. As used herein, the terms "fragment", "derivative" and "analog" refer to a polypeptide that substantially retains the function or activity of the RNA editing enzyme of the present invention. The polypeptide fragments, derivatives or analogues of the present invention can be (i) a polypeptide with one or more conservative or non-conservative amino acid residues (preferably conservative amino acid residues) substituted, or (ii) a polypeptide with substitution groups in one or more amino acid residues, or (iii) a polypeptide formed by fusion of an antigenic peptide with another compound (such as a compound that extends the half-life of the viral capsid protein mutant, such as polyethylene glycol), or (iv) an additional amino acid sequence is fused to this polypeptide sequence to form a polypeptide (fusion protein formed by fusion with leader sequence, secretory sequence or 6His and other tag sequences). According to the teachings herein, these fragments, derivatives and analogs fall within the scope of those skilled in the art.

A preferred type of active derivative means that compared with the amino acid sequence of Formula I, there are at most 3, preferably at most 2, and more preferably at most 1 amino acid replaced by an amino acid with close or similar properties to form a polypeptide. These conservative variant polypeptides are best produced according to Table A by performing amino acid substitutions.

**Table A**

| Initial residues | Representative substitution | Preferred substitution |
|---|---|---|
| Ala (A) | Val; Leu; Ile | Val |
| Arg (R) | Lys; Gln; Asn | Lvs |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lvs (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogs of the RNA editing enzyme of the present invention. The difference between these analogs and the polypeptide as shown in SEQ ID NO.: 8, 9 or 13 may be a difference in amino acid sequence, or a difference in modified form that does not affect the sequence, or both. Analogs also include analogs having residues different from natural L-amino acids (such as D-amino acids), and analogs having non-naturally occurring or synthetic amino acids (such as β, γ-amino acids). It should be understood that the polypeptide of the present invention is not limited to the representative polypeptides as exemplified above.

Modified (usually unchanged primary structure) forms include: chemically derived forms of peptides in vivo or in vitro, such as acetylation or carboxylation. Modifications also include glycosylation, such as those produced by glycosylation modification during the synthesis and processing or during further processing steps of peptides. This modification can be accomplished by exposing the peptides to an enzyme that performs glycosylation (such as mammalian glycosylase or deglycosylase). Modified forms also include sequences with phosphorylated amino acid residues (such as phosphotyrosine, phosphoserine, phosphothreonine). It also includes peptides that have been modified to improve their resistance to proteolysis or to optimize their solubility.

### Coding sequence

The present invention also relates to polynucleotides encoding RNA editing enzymes of the present invention.

The polynucleotide of the present invention may be in the form of DNA or RNA. DNA can be a coding strand or a non-coding strand. The full-length nucleotide sequence of the present invention or its fragments can usually be obtained by PCR amplification method, recombination method or artificial synthesis method. At present, the DNA sequence encoding the polypeptide (or fragment or derivative thereof) of the present invention can be obtained completely through chemical synthesis. The DNA sequence can then be introduced into various existing DNA molecules (or such as vectors) and cells known in the art.

The present invention also relates to a vector containing the polynucleotide of the present invention, and a host cell produced by genetic engineering using the vector or polypeptide coding sequence of the present invention. The aforementioned polynucleotides, vectors or host cells may be isolated.

As used herein, "isolated" refers to the separation of a substance from its original environment (if it is a natural substance, the original environment is the natural environment). For example, the polynucleotides and polypeptides in the natural state in living cells are not separated and purified, but the same polynucleotides or polypeptides are separated and purified if they are separated from other substances that co-exist in the natural state.

The polynucleotide of the present invention may be in the form of DNA or RNA. The form of DNA includes cDNA, genomic DNA or synthetic DNA. DNA can be single-stranded or double-stranded. DNA can be a coding strand or a non-coding strand.

The present invention also relates to variants of the above-mentioned polynucleotides, which encode protein fragments, analogs and derivatives having the same amino acid sequence as the present invention. The variants of this polynucleotide can be naturally occurring allelic variants or non-naturally occurring variants. These nucleotide variants include substitution variants, deletion variants and insertion variants. As known in the art, an allelic variant is an alternative form of a polynucleotide. It may be a substitution, deletion or insertion of one or more nucleotides, but the function of encoding the RNA editing enzyme of the present invention will not be substantially changed.

The full-length nucleotide sequence or fragments thereof encoding the fusion protein of the present invention can usually be obtained by PCR amplification method, recombinant method or artificial synthesis method. For the PCR amplification method, primers can be designed according to the published relevant nucleotide sequence, especially the open reading frame sequence, and using a commercially available cDNA library or a cDNA library prepared according to a conventional method known to those skilled in the art as a template, amplifying the relevant sequence. When the sequence is long, it is often necessary to perform two or more PCR amplifications, and then the amplified fragments are spliced together in the correct order.

Once the relevant sequence is obtained, the recombination method can be used to obtain the relevant sequence in large quantities. This is usually done by cloning it into a vector, then transferring it into a cell, and then isolating the relevant sequence from the proliferated host cell by conventional methods.

In addition, artificial synthesis methods can also be used to synthesize related sequences, especially when the fragment length is short. Usually, by first synthesizing multiple small fragments, and then ligating to obtain fragments with very long sequences.

The method of using PCR technology to amplify DNA/RNA is preferably used to obtain the gene of the present invention. The primers used for PCR can be appropriately selected according to the sequence information of the present invention disclosed herein and can be synthesized by conventional methods. The amplified DNA/RNA fragments can be separated and purified by conventional methods such as gel electrophoresis.

The present invention also relates to a vector containing the polynucleotide of the present invention, a host cell produced by genetic engineering using the vector or protein coding sequence of the present invention, and a method for expressing the RNA editing enzyme of the present invention on the NK cell by recombinant technology.

Through conventional recombinant DNA technology, the polynucleotide sequence of the present invention can be used to obtain NK cells expressing the RNA editing enzyme of the present invention. Generally, it includes the steps of: transducing the first expression cassette and/or the second expression cassette of the present invention into NK cells, so as to obtain the NK cells.

Methods well known to those skilled in the art can be used to construct an expression vector containing the coding DNA sequence of the RNA editing enzyme of the present invention and appropriate transcription/translation control signals. These methods include in vitro recombinant DNA technology, DNA synthesis technology, and in vivo recombination technology. The DNA sequence can be effectively linked to an appropriate promoter in the expression vector to guide mRNA synthesis. The expression vector also includes a ribosome binding site for translation initiation and a transcription terminator.

In addition, the expression vector preferably contains one or more selective marker genes to provide phenotypic traits for selection of transformed host cells, such as dihydrofolate reductase for eukaryotic cell culture, neomycin resistance, and green Fluorescent protein (GFP), or tetracycline or ampicillin resistance for E. coli.

A vector containing the above-mentioned appropriate DNA sequence and an appropriate promoter or control sequence can be used to transform an appropriate host cell so that it can express the protein.

The host cell can be a prokaryotic cell, such as a bacterial cell; or a lower eukaryotic cell, such as a yeast cell; or a higher eukaryotic cell, such as a mammalian cell. Representative examples include: Escherichia coli, Bacillus subtilis, Streptomyces bacterial cells; fungal cells such as Pichia pastoris, Saccharomyces cerevisiae cells; plant cells; Drosophila S2 or Sf9 insect cells; CHO, NS0, COS7, or 293 cells of animal cells and so on.

Transformation of host cells with recombinant DNA can be carried out by conventional techniques well known to those skilled in the art. When the host is a prokaryotic organism such as Escherichia coli, competent cells that can absorb DNA can be harvested after the exponential growth phase and treated with the CaCl₂ method. The steps used are well known in the art. Another method is to use MgCl₂. If necessary, the transformation can also be carried out by electroporation. When the host is a eukaryote, the following DNA transfection methods can be selected: calcium phosphate co-precipitation method, conventional mechanical methods such as microinjection, electroporation, liposome packaging, etc..

The obtained transformants can be cultured by conventional methods to express the protein encoded by the gene of the present invention. Depending on the host cell used, the medium used in the culture can be selected from various conventional mediums. The culture is carried out under conditions suitable for the growth of the host cell. After the host cells have grown to an appropriate cell density, the selected promoter is induced by a suitable method (such as temperature conversion or chemical induction), and the cells are cultured for a period of time.

The protein in the above method can be expressed in the cell or on the cell membrane, or secreted out of the cell. If necessary, the physical, chemical, and other properties can be used to separate and purify the protein through various separation methods. These methods are well known to those skilled in the art. Examples of these methods include, but are not limited to: conventional renaturation treatment, treatment with a protein precipitation agent (salting out method), centrifugation, bacteria broken through osmosis, ultra-treatment, ultra-centrifugation, molecular sieve chromatography (gel filtration), adsorption chromatography, ion exchange chromatography, high performance liquid chromatography (HPLC) and various other liquid chromatography techniques and combinations of these methods.

### Vector

The present invention also provides a vector containing the polynucleotide of the present invention, Vectors derived from retroviruses such as lentiviruses are suitable tools to achieve long-term gene transfer because they allow long-term, stable integration of the transgene and its propagation in daughter cells. Lentiviral vectors have advantages over vectors derived from oncogenic retroviruses such as murine leukemia virus because they can transduce non-proliferating cells, such as hepatocytes. They also have the advantage of low immunogenicity.

In a simple summary, usually by operably linking the expression cassette or nucleic acid sequence of the present invention to a promoter and incorporating it into an expression vector. The vector is suitable for replication and integration of eukaryotic cells. A typical cloning vector contains transcription and translation terminators, initial sequences, and promoters that can be used to regulate the expression of the desired nucleic acid sequence.

The expression construct of the present invention can also use standard gene delivery protocols for nucleic acid immunization and gene therapy. Methods of gene delivery are known in the art. See, for example, U.S. Patent Nos. 5,399,346, 5,580,859, 5,589,466, which are hereby incorporated by reference in their entirety. In another embodiment, the invention provides a gene therapy vector.

The expression cassette or nucleic acid sequence can be cloned into many types of vectors. For example, the expression cassette or nucleic acid sequence can be cloned into a vector including but not limited to plasmids, phagemids, phage derivatives, animal viruses, and cosmids. Specific vectors of interest include expression vectors, replication vectors, probe generation vectors, and sequencing vectors.

Further, the expression vector can be provided to the cell in the form of a viral vector. Viral vector technology is well known in the art and is described in, for example, Sambrook et al. (2001, Molecular Cloning: A Laboratory Manual, Cold Spring Harbor Laboratory, New York) and other virology and molecular biology manuals. Viruses that can be used as vectors include, but are not limited to, retrovirus, adenovirus, adeno-associated virus, herpes virus, and lentivirus. Generally, a suitable vector contains an origin of replication that functions in at least one organism, a promoter sequence, convenient restriction enzyme sites, and one or more selective markers (e.g., WO01/96584; WO01/29058; and U.S. Patent No. 6,326,193).

Many virus-based systems have been developed for gene transfer into mammalian cells. For example, retroviruses provide a convenient platform for gene delivery systems. The selected gene can be inserted into a vector and packaged into retroviral particles using techniques known in the art. The recombinant virus can then be isolated and delivered to target cells in vivo or ex vivo. Many retroviral systems are known in the art.

Additional promoter elements, such as enhancers, can regulate the frequency of transcription initiation. Generally, these are located in the 30-110 bp region upstream of the initiation site, although it has recently been shown that many promoters also contain functional elements downstream of the initiation site. The spacing between promoter elements is often flexible in order to maintain promoter function when the elements are inverted or moved relative to one another. In the thymidine kinase (tk) promoter, the spacing between promoter elements can be increased by 50 bp before the activity begins to decrease. Depending on the promoter, it appears that individual elements can act cooperatively or independently to initiate transcription.

An example of a suitable promoter is the early cytomegalovirus (CMV) promoter sequence. The promoter sequence is a strong constitutive promoter sequence capable of driving high-level expression of any polynucleotide sequence operably linked to it. Another example of a suitable promoter is elongation growth factor-1α (EF-1α). However, other constitutive promoter sequences can also be used, including but not limited to the simian virus 40 (SV40) early promoter, mouse breast cancer virus (MMTV), human immunodeficiency virus (HIV) long terminal repeat (LTR) promoter, MoMuLV promoter, avian leukemia virus promoter, Epstein-Barr virus immediate early promoter, Russ sarcoma virus promoter, and human gene promoters, such as but not limited to actin promoter, Myosin promoter, heme promoter and creatine kinase promoter. Further, the present invention should not be limited to the application of constitutive promoters. Inducible promoters are also considered part of the invention. The use of an inducible promoter provides a molecular switch that can turn on expression of a polynucleotide sequence operably linked to an inducible promoter when such expression is desired, or turn off expression when expression is undesirable. Examples of inducible promoters include, but are not limited to, metallothionein promoter, glucocorticoid promoter, progesterone promoter and tetracycline promoter.

The expression vector introduced into the cell may also contain either or both of a selective marker gene or a reporter gene to facilitate the identification and selection of the expression cell from the cell population seeking to be transfected or infected by the viral vector. In other aspects, the selective marker can be carried on a single piece of DNA and used in the co-transfection procedure. Both the selective marker and the reporter gene can be flanked by appropriate regulatory sequences so that they can be expressed in the host cell. Useful selective markers include, for example, antibiotic resistance genes such as neo and the like.

Reporter genes are used to identify potentially transfected cells and to evaluate the functionality of regulatory sequences. Generally, a reporter gene is a gene that does not exist in the recipient organism or tissue or is expressed by the recipient organism or tissue, and it encodes a polypeptide whose expression is clearly indicated by some easily detectable properties such as enzyme activity. After the DNA has been introduced into the recipient cell, the expression of the reporter gene is measured at an appropriate time. Suitable reporter genes may include genes encoding luciferase, β-galactosidase, chloramphenicol acetyltransferase, secreted alkaline phosphatase or green fluorescent protein genes (e.g., Ui-Tei et al., 2000FEBS Letters479: 79-82).

Physical methods for introducing polynucleotides into host cells include calcium phosphate precipitation, lipofection, particle bombardment, microinjection, electroporation, and so on. Methods of producing cells including vectors and/or exogenous nucleic acids are well known in the art. See, for example, Sambrook et al. (2001, Molecular Cloning: A Laboratory Manual, Cold Spring Harbor Laboratory, New York). The preferred method for introducing polynucleotides into host cells is calcium phosphate transfection.

Biological methods for introducing polynucleotides into host cells include the use of DNA and RNA vectors. Viral vectors, especially retroviral vectors, have become the most widely used method of inserting genes into mammalian, such as human cells. Other viral vectors can be derived from lentivirus, poxvirus, herpes simplex virus I, adenovirus, adeno-associated virus, and so on. See, for example, U.S. Patent Nos. 5,350,674 and 5,585,362.

Chemical means for introducing polynucleotides into host cells include colloidal dispersion systems, such as macromolecular complexes, nanocapsules, microspheres, and beads; and lipid-based systems, including oil-in-water emulsions, micelles, mixed micelles, and lipidosome. Exemplary colloidal systems used as delivery vehicles in vitro and in vivo are liposomes (e.g., artificial membrane vesicles).

Where a non-viral delivery system is used, an exemplary delivery vehicle is a liposome. Consider using lipid formulations to introduce nucleic acids into host cells (in vitro, ex vivo, or in vivo). In another aspect, the nucleic acid can be associated with lipids. Lipid-associated nucleic acids can be encapsulated in the aqueous interior of liposomes, dispersed in the lipid bilayer of liposomes, and attached to the liposome via a linking molecule associated with both the liposome and the oligonucleotide, trapped in liposomes, complexed with liposomes, dispersed in a solution containing lipids, mixed with lipids, combined with lipids, contained in lipids as a suspension, contained in micelles or complexed with micelles, or otherwise associated with lipids. The lipid, lipid/DNA or lipid/expression vector associated with the composition is not limited to any specific structure in the solution. For example, they may exist in a bilayer structure, as micelles or have a "collapsed" structure. They can also simply be dispersed in the solution, possibly forming aggregates of uneven size or shape. Lipids are fatty substances, which can be naturally occurring or synthetic lipids. For example, lipids include fat droplets, which occur naturally in the cytoplasm and in such compounds containing long-chain aliphatic hydrocarbons and their derivatives such as fatty acids, alcohols, amines, amino alcohols, and aldehydes.

### preparation

The present invention provides the preparation according to the fifth aspect of the present invention. In one embodiment, the preparation is a liquid formulation. Preferably, the preparation is an injection.

In one embodiment, the preparation may include buffers such as neutral buffered saline, sulfate buffered saline, etc.; carbohydrates such as glucose, mannose, sucrose or dextran, mannitol; protein; polypeptides or amino acids such as glycine; Antioxidant; Chelating agent such as EDTA or glutathione; Adjuvant (for example, aluminum hydroxide); and Preservative.

The technical solution of the present invention has the following beneficial effects:
(1) The RNA editing enzyme of the present invention is a single-component protease that does not contain any RNA components and is composed of endogenous human protein sequences. Therefore, these engineered proteins have lower immunogenicity and system complexity than the CRISPR-Cas system in gene therapy. Moreover, the system is more flexible and safer than DNA editing.
(2) The RNA editing enzyme of the present invention has a low off-target rate and high editing efficiency.
(3) The RNA editing enzyme of the present invention has high editing precision and can realize single-base gene editing.
(4) The RNA editing enzyme of the present invention is a protease, which can be targeted to the organelle or nucleus to function through organelle localization signals, for example, a nuclear localization signal NLS is connected to the N-terminal or C-terminal of the RNA editing enzyme, localize the editing enzyme to the nucleus to function, or connect a mitochondrial localization signal MLS to localize the enzyme to the mitochondria to play an editing function.

The present invention will be further explained below in conjunction with specific implementations. It should be understood that these embodiments are only used to illustrate the present invention and not to limit the scope of the present invention. The experimental methods without specific conditions in the following examples are usually based on conventional conditions, such as the conditions described in Sambrook et al., Molecular Cloning: Laboratory Manual (New York: Cold Spring Harbor Laboratory Press, 1989), or according to manufacturing The conditions suggested by the manufacturer. Unless otherwise stated, percentages and parts are calculated by weight.

### Experimental method

The deaminated catalytic domains of members of the RNA adenosine deaminase ADAR family and members of the RNA cytidine deaminase APOBEC family were cloned and fused with the RNA binding protein PUF to form two different RNA editing enzymes PARSE and APRSE. According to the above design, it was verified that PARSE and APRSE RNA editing enzymes can edit RNA at the cellular level. Detecting the editing efficiency and precision of these enzymes, and analyzing the off-target effects of these enzymes at the transcriptome level. The constructed RNA editing enzyme is used to repair some single-base pathogenic point mutations, which are used to repair the pathogenic point mutations of GRIA2, COL3A1, DMD, EZH2, SCN1A and SCN5A genes.

Specifically, it includes the following steps:
1. Based on the existing RNA binding protein PUF, the dADAR, ADAR1 and ADAR2 genes encoding the ADAR protein were cloned separately using molecular cloning technology, and then these two genes were spliced together to form a new fusion gene. The PARSE protein was encoded by the new fusion gene, constructing and forming different A-to-I RNA site-directed editing enzymes. Using molecular cloning technology to fuse different RNA cytidine deaminase (human apobec1, human apobec3a, human3b, human3c, human3d, human3g, human3h, humanAID, mouse APOBEC1, mouse APOBEC3A, mouse AID, Rat APOBEC1) to PUF, constructing and forming different C-to-U RNA site-directed editing enzymes.
2. Detecting the editing activity of these new RNA editing enzymes at the cellular level, transferring the PARSE RNA editing enzyme and the GFP reporter gene plasmid into the cells at the same time, and after 48 hours, the RNA of the GFP reporter gene was collected and sequenced to detect RNA editing events.
3. Detecting the editing activity of these new RNA editing enzymes at the cellular level, transferring the APRSE RNA editing enzyme and the GFP reporter gene plasmid into the cells at the same time, and after 48 hours, the RNA of the GFP reporter gene was collected and sequenced to detect RNA editing events.
4. Detecting and analyzing the efficiency, precision and off-target rate of RNA editing enzymes through RNA seq high-throughput sequencing technology.
5. Using the constructed RNA editing enzyme to repair some single-base disease-causing mutations, and using PARSE to repair the pathogenic point mutations such as GRIA2, COL3A1 and DMD.
6. Using the constructed RNA editing enzyme to repair some single-base disease-causing mutations, and using APRSE to repair the pathogenic point mutations of genes such as EZH2, SCN1A and SCN5A.

### Example 1

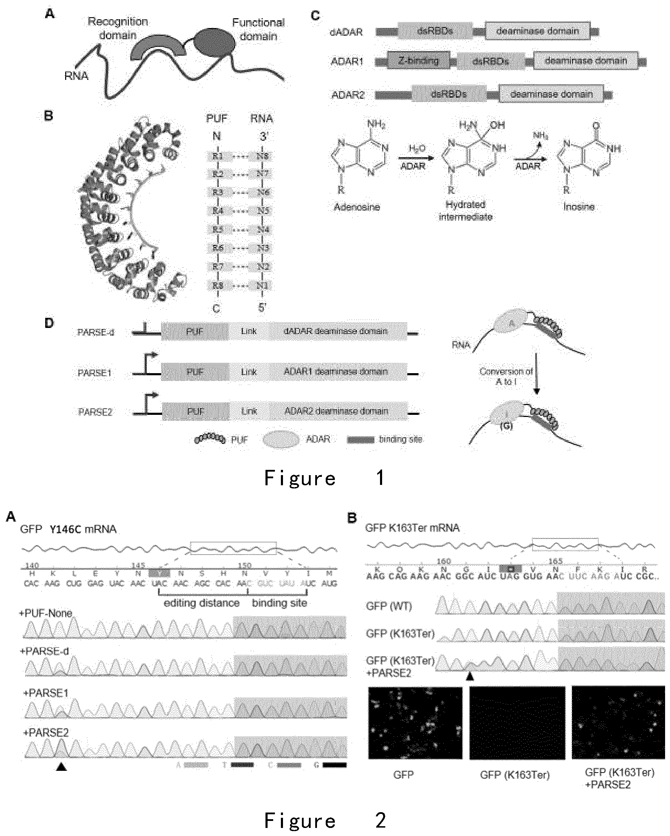

ADAR is an adenosine editing enzyme that can catalyze the deamination of RNA adenosine to form inosine (adenosine-to-inosine, A-to-I). The ADAR catalytic domains from three different sources were cloned and fused with the RNA binding protein PUF through linker, and three different artificial RNA editing enzymes (RNA editase) were developed and the system was named artificial PUF-ADAR RNA sequence editors (PARSE-d, PARSE1, PARSE2) (Figure 1, A-D)

Detecting the editing activity of the three RNA editing enzymes, PARSE-d, PARSE1 and PARSE2 at the cell level, transferring the PARSE RNA editing enzyme and the GFP reporter gene plasmid into the cells at the same time, and after 48h, the RNA of the GFP reporter gene was collected and sequenced to detect RNA editing events, proving that PARSE-d, PARSE1 and PARSE2 have the ability to edit target RNA efficiently (Figure 2A). By constructing a GFP mRNA containing an early stop codon, editing from A to G at a specific site through PARSE, thereby restoring the expression of the GFP gene, proving that PARSE has high-precision fixed-point editing capabilities (Figure 2B).

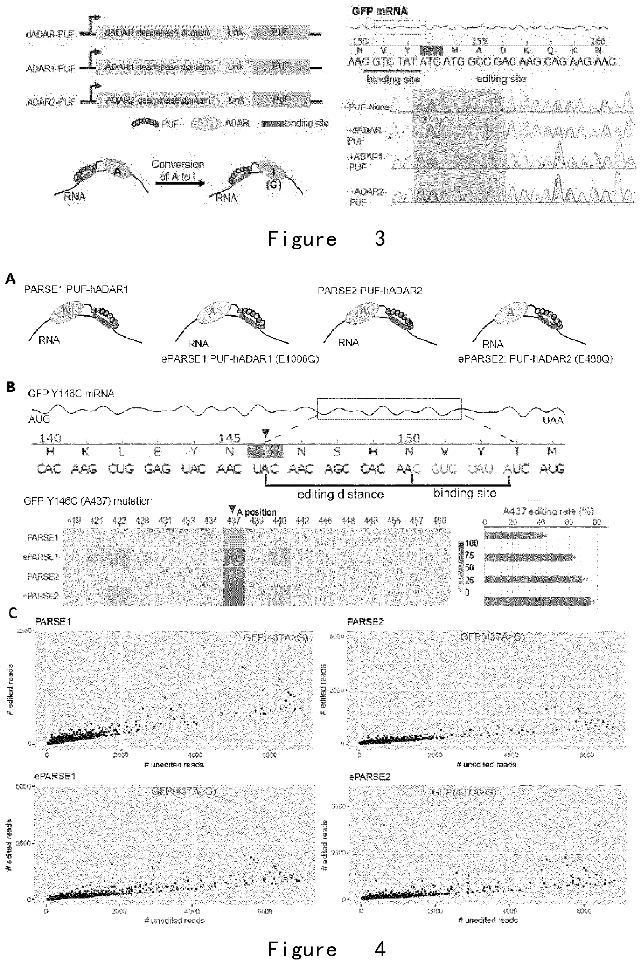

The ADAR catalytic domains from three different sources were cloned, and fused through linker with RNA binding protein PUF, ADAR was placed at the N end of the newly designed fusion protein, and PUF was placed at the C end of the newly designed fusion protein to form a new RNA editing enzyme with new ADAR catalytic domain first and PUF RNA binding protein behind, using this newly synthesized RNA editing enzyme to edit target RNA, and no editing events were detected (Figure 3). This proves that ADAR-dependent RNA editing enzymes have strict requirements on the position of RNA binding proteins, and the RNA editing enzyme of the present invention has excellent RNA editing activity.

### Example 2

By optimizing the ADAR catalytic domain to improve the editing efficiency of ADAR on target RNA, and through the RNA seq high-throughput sequencing technology, the efficiency, accuracy and off-target rate of RNA editing enzymes can be detected and analyzed. A new point mutation was introduced into the catalytic domains of ADAR1 and ADAR2 to improve the editing efficiency of ADAR (Figure 4A). The RNA seq high-throughput sequencing technology was used to detect and analyze the efficiency and accuracy of RNA editing enzymes. It has been found that PARSE1, ePARSE1, PARSE2, and ePARSE2 can all edit target RNA with efficiencies of 42%, 65%, 67%, and 78%, respectively (Figure 4B). The analysis of RNA-seq results shows that PARSE editing has a certain degree of off-target rate, but compared with the editing efficiency of the target site, the off-target efficiency is lower. The off-target rate can be reduced by reducing the amount of PARSE transfection in the later optimization process (Figure 4C).

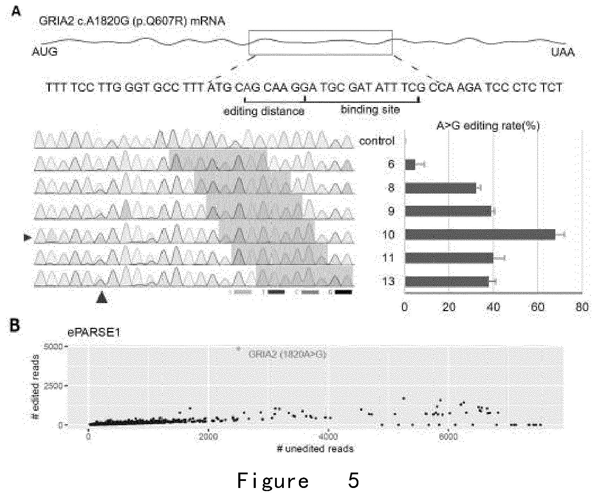

Using the constructed ePARSE1 to repair the pathogenic editing site of GRIA2 gene. GRIA2 is a subunit of calcium channel protein. The mutation of the position 607 amino acid of the protein will cause the calcium channel protein to be unable to close, resulting in a pathogenic phenotype. Using ePARSE1 to perform site-specific repair on this site, the results show that the repair efficiency is 68%, that is, the repair of the pathogenic point mutation of GRIA2 (p.Q607R) can be completed at the cellular level, providing a powerful tool for further treatment of this gene mutation (Figure 5A), and the off-target efficiency of this site is low (Figure 5B), showing a very good application prospect of ePARSE1 to treat this disease.

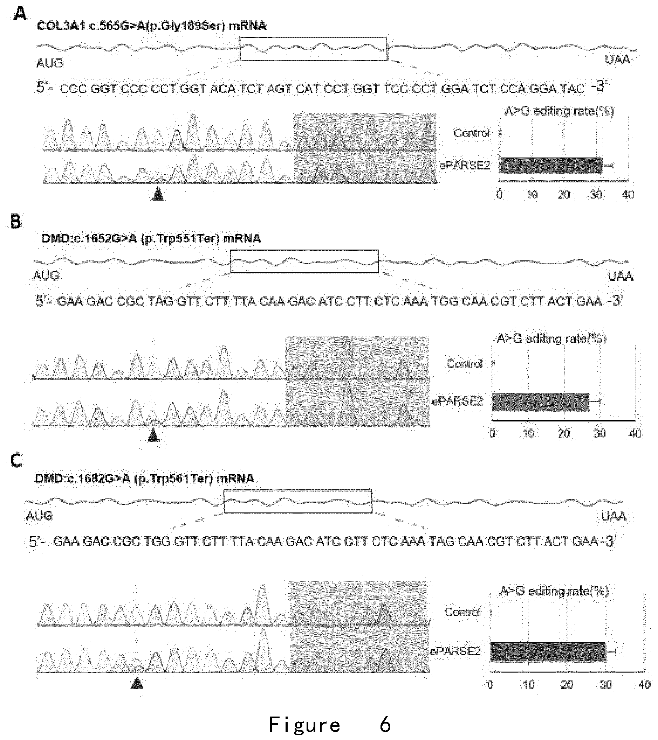

The constructed ePARSE2 was used for site-specific repair of the pathogenic point mutations of the COL3A1 and DMD genes. The results show that the repair efficiency is 33%, 25%, and 30%, respectively, that is, the site-specific repair of the pathogenic point mutations of COL3A1 and DMD genes can be completed at the cellular level, providing a powerful tool for further treatment of this gene mutation (Figure 6A, 6B, 6C), showing the good application prospects of ePARSE2 in the treatment of this type of disease.

### Example 3

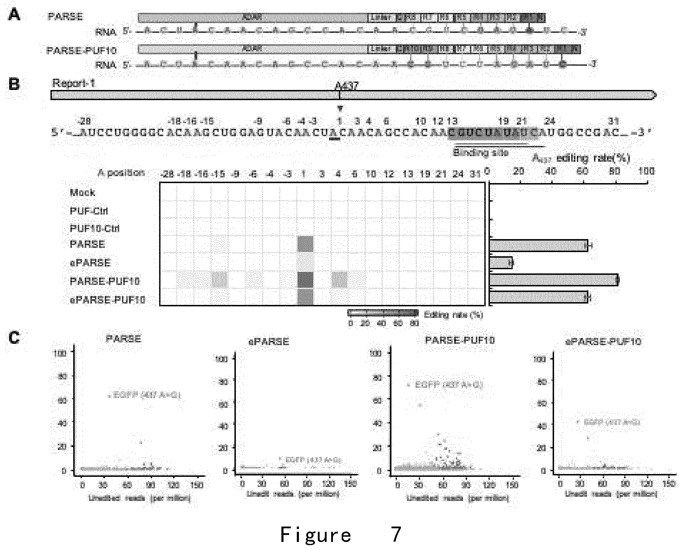

Optimize the RNA binding protein PUF to improve the accuracy of PARSE editing RNA and reduce the off-target rate. By optimizing the PUF domain, the PUF8 that recognizes 8 bases was optimized to the PUF10 that recognizes and binds to ten bases (Figure 7, A-C), this optimized design does not reduce the editing efficiency of RNA target sites, and as the number of PUF recognition and binding bases is increased, this strategy reduces the off-target rate of PARSE by 10 times, effectively reducing off-target effects and has the potential for further optimization, PUF can be optimized to recognize and bind 12 bases, 16 bases, or even longer, which can greatly expand the application range of PARSE.

### Example 4

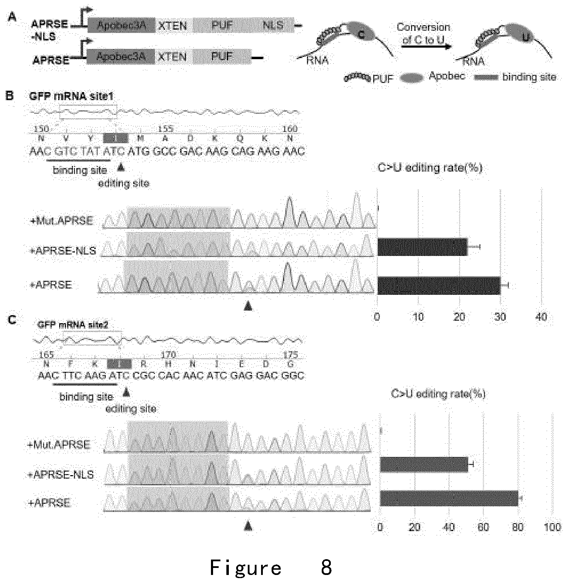

APOBECs can catalyze the nucleotide editing of cytidine-to-uridine (C-to-U), and the catalytic domains of RNA cytidine deaminase from various sources of APOBEC family(human apobec1, human apobec3a, human3b, human3c , human3d, human3g, human3h, humanAID, mouse APOBEC1, mouse APOBEC3A, mouse AID, Rat APOBEC 1) were cloned and fused with the RNA binding protein PUF through linker to form a variety of new C to U RNA site-directed editing enzymes, the system is named artificial APOBEC-PUF RNA sequence editors (APRSE), and is further subdivided into APRSE-NLS that can enter the nucleus and APRSE that is expressed in the cytoplasm. The figure below takes Apobec3A as an example (Figure 8A).

Detecting the editing activity of the two RNA editing enzymes APRSE-NLS and APRSE at the cell level, transferring the APRSE RNA editing enzyme and the GFP reporter gene plasmid into the cells at the same time, and after 48h, the RNA of the GFP reporter gene was collected and sequenced to detect RNA editing events. It is proved that APRSE-NLS and APRSE have the ability to edit target RNA efficiently (Figure 8B, 8C). The results show that APRSE has high-precision fixed-point editing ability, which can directly edit the second base downstream of the APRSE binding site with high precision and efficiency, the editing efficiency of different editing sites is between 30% and 80%.

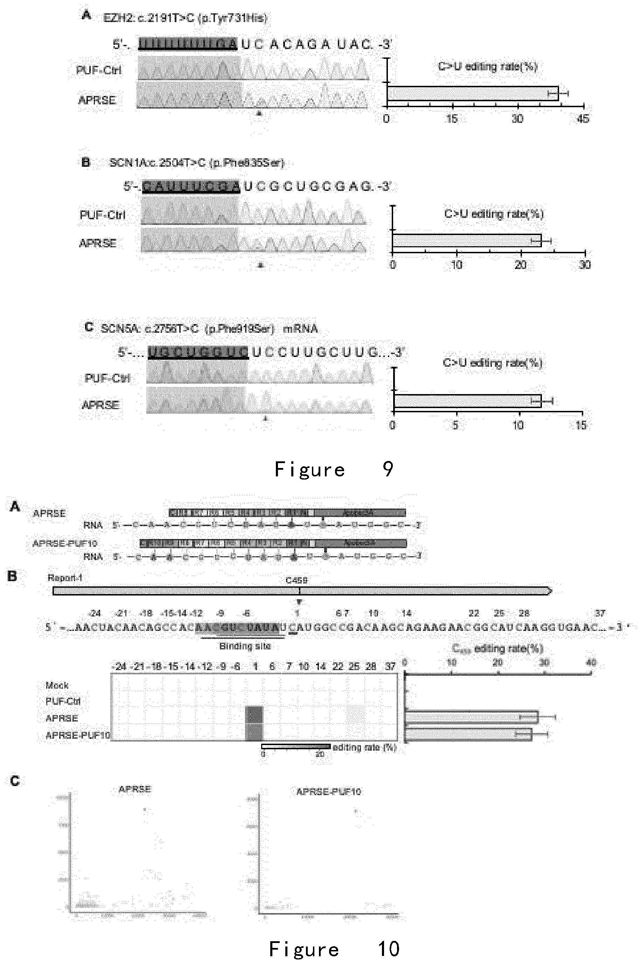

The constructed APRSE was used for site-specific repair of the pathogenic point mutations of EZH2, SCN1A and SCN5A genes. The results show that the repair efficiency is 39%, 23%, and 12% respectively, that is, the site-specific repair of the pathogenic point mutations of EZH2, SCN1A and SCN5A genes can be completed at the cellular level, providing a powerful tool for further treatment of this gene mutation (Figures 9A, 9B, 9C), showing the good application prospects of APRSE to treat this type of disease.

### Example 5

Optimizing the RNA-binding protein PUF to improve the accuracy of APRSE's RNA editing and reduce off-target efficiency. By optimizing the PUF domain, the PUF8 that recognizes 8 bases is optimized to the PUF10 that recognizes and binds to ten bases (Figure 10, A-C), this optimized design does not reduce the editing efficiency of RNA target sites, and with increasing the number of PUF recognition and binding bases, this strategy reduces the off-target effect of APRSE by 12 times, effectively reducing off-target effects, and has the potential for further optimization, PUF can be optimized to recognize and bind 12 bases, 16 bases, or even longer, which can greatly expand the application range of APRSE.

### Discussion

Compared with direct specific editing and manipulation of DNA, specific manipulation of RNA is reversible and has greater flexibility. RNA-targeted gene therapy can effectively avoid the shortcomings of DNA gene therapy. Therefore, the manipulation of genes at the RNA level has better controllability and safety, making this type of gene therapy more conducive to the transformation of basic research into clinical practice.

The current base editing at the RNA level mainly includes REPAIR based on CRISPR-Cas13 and the method of recruiting cell endogenous RNA adenosine deaminase ADAR through oligonucleotide fragments for RNA editing. REPAIR based on CRISPR-Cas13 also has the same problems as the above-mentioned DNA editing, including the system is too large, editing efficiency and editing accuracy are low, and it is easy to cause immune responses and other problems; Based on oligonucleotide fragments recruiting cell endogenous RNA adenosine deaminase for RNA editing, the current application range is too small, there are also difficulties in delivery and single efficacy, and the system is more complicated.

The present inventors fused the RNA recognition domain with the functioning utility domain (effector domain) to form a new functional protein, which specifically targets the target RNA through the recognition domain and utilizes the utility domain to perform RNA editing, to eliminate pathogenic RNA by correcting the wrong point mutations in the DNA. The artificially constructed RNA editing enzyme is a single-component protease that does not contain any RNA components and is composed of endogenous human protein sequences. Therefore, these engineered proteins have lower immunogenicity and system complexity than the CRISPR-Cas system in gene therapy. Moreover, the system is more flexible and safer than DNA editing.

In addition, because the artificial RNA editing enzyme based on a single protein can connect different cell location sequences to locate in different subcellular structures (including nucleus, cytoplasm, mitochondria, chloroplasts, etc.), artificial RNA editing enzymes can specifically edit RNA in different subcellular structures. Taking mitochondria as an example, there is currently no effective editing method for mitochondrial genes, so that artificial RNA editing enzymes can have an irreplaceable role in mitochondrial gene manipulation.

Therefore, constructing modular artificial RNA-binding proteins through synthetic biological means, and fusing RNA editing proteins to RNA-binding proteins, so as to specifically control the editing of targeted RNAs, is a new treatment idea for targeted RNAs. Since the artificially designed PUF factor can be reprogrammed to recognize almost any 8-nucleotide sequence, it can theoretically be used to edit any given RNA transcript. It is hoped that through the application of this system, some disease-related mutations can be targeted and edited. This system provides useful tools for targeted editing of RNA in human cells, and hopefully treats some diseases caused by nucleic acid mutations. Moreover, the artificially constructed PUF-Factor is a simple enzyme that does not contain any RNA components. It is composed of endogenous human protein sequences. These engineered proteins may be a simpler and more practical alternative than the CRISPR-Cas system in gene therapy.

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. An RNA editing enzyme, comprising:
(a) a RNA recognition domain, the RNA recognition domain is used to recognize the RNA recognition sequence of the RNA sequence to be edited, and bind to the RNA recognition sequence;
(b) a utility domain, which is used for nucleotide editing of the RNA sequence to be edited;
wherein, the RNA recognition domain and the utility binding domain are operably linked.

2. The RNA editing enzyme of claim 1, wherein the utility domain is selected from the group consisting of the deamination catalytic domain of ADAR family members, the deamination catalytic domain of APOBEC family members, RNA methylase, RNA demethylase, added uracil synthase, and a combination thereof.

3. The RNA editing enzyme of claim 1, wherein the RNA recognition domain contains n recognition units, and each recognition unit is used to recognize an RNA base, wherein n is a positive integer of 5-30.

4. The RNA editing enzyme of claim 1, wherein the RNA editing enzyme further includes one or more elements selected from the group consisting of linker peptide, tag sequence, signal peptide sequence, location peptide sequence, and a combination thereof.

5. The RNA editing enzyme of claim 1, wherein the RNA editing enzyme includes an RNA recognition domain and a utility domain, as well as an optional linker peptide, tag sequence, signal peptide sequence and/or location peptide sequence.

6. The RNA editing enzyme of claim 1, wherein the structure of the RNA editing enzyme is shown in any one of the following Formula I to formula IV:
D-L2-A-L1-B (I);
D-L2-B-L1-A (II);
A-L1-B-L2-D (III);
B-L1-A-L2-D (IV);
wherein each "-" is independently a linker peptide or a peptide bond;
A is a RNA recognition domain;
B is a utility domain;
L1 and L2 is each independently none or a linker peptide;
D is none or a location peptide.

7. The RNA editing enzyme of claim 2, wherein the ADAR family member includes: dADAR, ADAR1, ADAR2, TadA, and a combination thereof.

8. An isolated polynucleotide which encodes the RNA editing enzyme of claim 1.

9. A vector, which comprises the polynucleotide of claim 8.

10. Use of the RNA editing enzyme of claim 1, or the polynucleotide of claim 8, or the vector of claim 9, the host cell for the preparation of
(a) a drug or preparation for gene therapy; and/or
(b) a reagent for editing RNA.
